(19) Europäisches Patentamt
European Patent Office
Office européen des brevets

(11) Publication number: **0 409 625 A1**

# (12) EUROPEAN PATENT APPLICATION

(21) Application number: 90307915.0

(22) Date of filing: **19.07.90**

(51) Int. Cl.⁵: **C12N 15/29**, C12N 15/82, A01H 5/00, C12N 5/10

(30) Priority: **19.07.89 US 382176**

(43) Date of publication of application:
**23.01.91 Bulletin 91/04**

(84) Designated Contracting States:
**AT BE CH DE DK ES FR GB GR IT LI LU NL SE**

(71) Applicant: **CALGENE, INC.**
**1920 Fifth Street Suite F.**
**Davis California 95616(US)**

(72) Inventor: **Houck, Cathrine M.**
**172 Carmel Court**
**Vacaville, California 95688(US)**

(74) Representative: **Paget, Hugh Charles Edward**
**et al**
**MEWBURN ELLIS 2 Cursitor Street**
**London EC4A 1BQ(GB)**

(54) **Fruit-specific transcriptional factors.**

(57) Fruit-specific regulatory regions are identified employing cDNA screening. The resulting fruit-specific regulatory regions are manipulated for use with foreign sequences for introduction into plant cells to provide transformed plants having fruit with a modified phenotypic property. The invention is exemplified with a tomato fruit-specific promoter which is active throughout the stages of fruit ripening.

EP 0 409 625 A1

# FRUIT-SPECIFIC TRANSCRIPTIONAL FACTORS

## INTRODUCTION

### Technical Field

This invention relates to DNA expression cassettes capable of directing fruit-specific expression of *in vitro* constructed expression cassettes in plants. The invention is exemplified by promoters useful in fruit-specific transcription in a tomato plant.

### Background

Manipulation of plants has proven to be significantly more difficult than manipulation of prokaryotes and mammalian hosts. As compared to prokaryotes and mammalian cells, much less was known about the biochemistry and cell biology of plant cells and plants. The ability to transform plant cells and regenerate plants is unique to flora since other differentiated species provide readily available transformable germ cells which may be fertilized and introduced into the live host for fetal development to a mature fetus. There has been substantial interest in modifying the ovum with inducible transcriptional initiation regions to afford inducible transcription and expression of the gene introduced into the ovum, rather than constitutive expression which would result in expression throughout the fetus.

For plants, it also is frequently desirable to be able to control expression at a particular stage in the growth of the plant or in a particular plant part. During the various stages of the growth of the plant, and as to the various components of the plant, it will frequently be desirable to direct the effect of the construct introduced into the entire plant or a particular part and/or to a particular stage of differentiation of the plant cell. For thia purpose, regulatory sequences are required which afford the desired initiation of transcription in the appropriate cell types and/or at the appropriate time of plant development, without having serious detrimental effects on plant development and productivity.

It is therefore of interest to be able to isolate sequences which can be manipulated to provide the desired regulation of transcription in a plant cell host during the growing cycle of the plant. One aspect of this interest is the ability to change the phenotype of fruit, so as to provide fruit which will have improved aspects for storage, handling, cooking, organoleptic properties, freezing, nutritional value, and the like.

### Relevant Literature

Plant promoter regions generally contain all elements involved in their expression pattern confined to a small portion of the 5′- region. Examples are the rbc S3A gene or pea in which the sequences essential for proper light regulation and tissue specific expression are contained within 400 bp of the 5′- region (Kuhlemeier, et al., *Plant Cell* (1989) 1:471-478) and the nopaline synthase gene in which tissue specific and developmentally regulated expression is contained within 160 bp of the 5′- region (Mitra and An, *Mol. Gen. Genet.* (1989) 215:294-299). A more than one kpb long 5′-region of the phytohemagglutinin L gene of the common bean regulates proper seed specific expression patterns (Riggs, et al., *Plant Cell* (1989) 1:609-621). A 1500 bp 5′ - region of the sun flower helianthinin gene HaG3A regulates seed-specific expression of this gene (Jordano, et al., *The Plant Cell* (1989) 1:855-866). Fruit-specific expression of two ethylene inducible genes, E4 and E8, has been reported (See Cordes, et al., *Plant Cell* (1989) 1:1025-1034 and Deikman and Fisher, *EMBO J.* (1988) 7:3315-3320). Sub-domains of the CaMV 35S promoter have been mapped (Benfey, *et al., EMBO Journal* (1990) 9:1677-1684 and 1685-1696). Likewise, sub-domains of the pSSU promoter have also been mapped. Gilmartin, *et al., Plant Cell* (1990) 2:369-378.

cDNA clones from tomato displaying differential expression during fruit development have been isolated and characterized (Mansson *et al., Mol. Gen. Genet.* (1985) 200:356-361; Slater *et al., Plant Mol. Biol.* - (1985) 5:137-147). The studies have focused primarily on mRNAs which accumulate during fruit ripening. One of the proteins encoded by the ripening-specific cDNAs has been identified as polygalacturonase (Slater *et al., Plant Mol. Biol.* (1985) 5:137-147). A cDNA clone which encodes tomato polygalacturonase has been sequenced. Grierson *et al., Nucleic Acids Research* (1986) 14:8395-8603. The concentration of polygalacturonase mRNA increases 2000-fold between the immature green and red-ripe stages of fruit development. This suggests that expression of the enzyme is regulated by the specific mRNA concentration which in turn is regulated by an increase in transcription. Della Penna *et al., Proc. Natl. Acad. Sci. USA* - (1986) 83:6420-6424. Mature plastic mRNA for psbA (one of the components of photosystem II) reaches its highest level late in fruit development, whereas after the onset or ripening, plastid mRNAs for other components of photosystem I and II decline to nondetectable levels in chromoplasts. Piechulla *et al., Plant Mol. Biol.* (1986) 7:367-376.

Other studies have focused on cDNAs encoding genes under inducible regulation, e.g. proteinase inhibitors which are expressed in response to wounding in tomato (Graham *et al., J. Biol. Chem.* (1985) 260:6555- 6560; Graham *et al., J. Biol. Chem.* (1985) 260:6561-6564) and on mRNAs correlated with ethylene synthesis in ripening fruit and leaves after wounding. Smith *et al., Planta* (1986) 168:94-100.

Leaf disc transformation of cultivated tomato is described by McCormick, *et al., Plant Cell Reports* - (1986) 5:81-89.

## SUMMARY OF THE INVENTION

Novel DNA constructions are provided comprising a fruit-specific promoter, particularly one active beginning at or shortly after anthesis or beginning at the breaker stage, joined to a DNA sequence of interest and a transcriptional termination region. The expression cassette is introduced into a plant cell host for integration into the host cell genome. The host cell is then grown to express the DNA sequence of interest. High levels of expression products can be achieved during formation and/or ripening of fruit. The expression cassettes find use in expressing a DNA sequence of interest preferentially in fruit. Additionally, DNA constructs comprising a fruit specific promoter find use as probes.

## BRIEF DESCRIPTION OF THE DRAWINGS

Figure 1 shows the nucleotide sequence of the cDNA clones pCGN1299 (2All) and pCGN1298 (3HII). The amino acid sequence of the polypeptide encoded by the open reading frame is also indicated.

Figure 2 is a comparison of 2All to pea storage proteins and other abundant storage proteins:

(a) 2All (residues 33-46) is compared to PAlb and the reactive site sequences of some protease inhibitors, PAlb (residues 6-23), chick pea inhibitor (residues 11-23), lima bean inhibitor (residues 23-35), human I-antitrypsin reactive site peptide. The arrow indicates the reactive site.

(b) is a comparison of the amino terminal sequence of 2All with the amino termini of a range of seed proteins. The data have been modified or deletions introduced to maximize homology; conserved residues are shown boxed. The seqeunces are from the following sources: PAlb; barley chloroform/methanol-soluble protein d; wheat albumin; wheat α-amylase inhibitor 0.28; millet bi-functional inhibitor; castor bean 2S small subunit; and napin small subunit.

Figure 3 shows the complete sequence of the 2All genomic DNA cloned into pCGN1273 from the *Xhol* site (position 1 at the 5' end) to the *Eco*RI site position 4654).

Figure 4 is a schematic diagram of the construction of the binary plasmid pCGN783; (a) through (f) refer to the plasmid constructions in Example 6.1.

Figure 5 shows the nucleotide sequence of a polygalacturonase (PG) genomic clone.

Figure 6 is a map of plasmid pCGN1288.

Figure 7 shows 2All genomic constructs. The upper line shows a map of the 2All genomic clone. The transcriptional start site, the polyadenylation site, the start (ATG) and stop (TGA) sites and the position of the intron are indicated. The hatched region indicates the portion of the genomic clone that was used to make the tagged 2All constructions. The bottom portion shows the regions used to construct the 2All cassettes including the synthetic oligonucleotide used to insert restriction sites and reconstruct the 3' end.

Figure 8 shows examples of 2All cassettes. Four versions of the 2All cassette are shown. They differ only in the flanking poly-linker regions and in the antibiotic resistance marker on the plasmid.

Figure 9 shows 2All-Gus constructions. The 2All-Gus construction is shown before and after transfer to a binary plasmid. Both orientations of the 2All-Gus construct in the binary plasmid are in cocultivation.

Figure 10 shows 2All truncated promoter constructions. The 2All-Gus constructions containing truncated promoters and alternate 3′ ends are shown prior to transfer to the binary plasmid.

Figure 11 shows a schematic map of the 2All 5′-region. The fragments of the 2All 5′-region obtained by the polymerase chain reaction are shown, with their length in bp indicated. Also indicated are the mRNA start side (+1). The TATA-box and the start codon of the 2All coding region.

Figure 12 shows gel retardation experiments. 100 ng of labeled fragment was incubated in the presence of poly dl/dC DNA with nuclear extracts isolated from leaf or fruit tissue (see methods).

(a) binding of leaf and fruit nuclear extracts to fragments 500, 5A, 5B and K.

(b) competition experiments for the binding of fruit nuclear proteins to fragment G. Competition shown with fragments G and H. The free DNA fragment (F) and the protein bound DNA/protein complex (B) are indicated.

(c) competition experiments for the binding of fruit nuclear proteins to fragment K. Competition shown with fragments K and G. The free DNA fragment (F) and the protein bound DNA/protein complex (B) are indicated.

Figure 13 shows protein binding sites within the 2A11 5′-region. Schematic map of the 2All 5′-region indicating the binding sites of the fruit specific and general binding factors, detected in the gel retardation experiments. Fragments C and E bind the same or similar proteins, as indicated in the figure.

Figure 14 shows Gus activities in 2All/35S plants. The Gus activities in pMol/mg protein/min measured in fruit tissue of all individual plants are shown in the bar-diagrams above the schematic maps of the 2All 5′-region, while the gus activities measured in the leaf tissues are shown in the bar-diagrams below these schematic maps. The results of the different constructs are shown at their relative positions to the schematic maps. The construct number and the fragment of the 2All 5′-region present in this construct are printed in the diagrams. The two last bars in each diagram are the negative control (UC82B; 30 in fruit and 5 in leaf) and the positive control containing the double 35S gus construct (20000 in fruit and 3000 in leaf). The control diagrams represent the plants containing the truncated CaMV 35S promoter without an insert (pCGN31490) and with the CaMV 35S enhancer inserted in front of it (pCGN3141).

Figure 15 shows the location of the enhancer elements in the 2All 5′-region. A schematic map of the 2All 5′-region is shown, with the sites of the putative fruit specific and general or leaf specific enhancer elements, as detected in the activity experiments with the 2All/35S hybrid promoters. The per fragments used in these experiments are indicated below the mpa.

Figure 16 shows the location of the 2All negative and positive regulator elements. Schematic map of the 2All 5′-region with the relative positions of the putative negative and positive regulatory elements indicated. The positive and fruit specific enhancer element ware shown as F-1 to F-5, the general or leaf specific elements as E-1 to E-3 and the silencer elements as S-1 to S-3.

## DESCRIPTION OF THE SPECIFIC EMBODIMENTS

In accordance with the subject invention, DNA constructs are provided which provide for modification of plant phenotype during fruit maturation and ripening. by use of a regulated transcriptional initiation region associated with fruit development and ripening. Expression cassettes can be prepared which comprise, a promoter from a gene associated with fruit development and ripening, a DNA sequence of interest and a transcriptional termination region. Transformation of the expression cassette into a host plant cell will provide for modification of the phenotype of fruit in a plant generated from the transformed cell. Desirably, the expression cassette will contain sequences which allow for integration of the transcriptional cassette into the genome of the plant host cell. The cassette additionally may include a multiple cloning site downstream from the fruit related promoter, so that the integration construct may be employed to express a variety of sequences preferentially in fruit.

The expression cassette will include in the 5′-3′ direction of transcription, a transcriptional and translational initiation region associated with gene expression in fruit-tissue, a DNA sequence of interest, and a transcriptional and translational termination region functional in plants. One or more introns may be also be present. Of particular interest are initiation regions (also sometimes referred to as "promoters") which provide for preferential or at least substantially specific expression in fruit as compared to other tissue such as leaf, stems or roots. By "at least substantially" is intended that expression in fruit is greater than about 100 fold that in other tissue. By "fruit" is intended the ripened ovary wall of a flower and any other closely associated parts (See Weirer, T.E. *et al., ed., Botany: An Introduction to Plant Biology* (6th Ed.) (John Wiley & Sons, 1982); Toothill & Backmore, *eds., The Facts on a File Dictionary of Botany* - (Market Home Books Ltd., 1984).

The ripening stages of the tomato may be broken down into mature green, breaker, turning, pink, light red and red. Desirably, the transcriptional initiation region maintains its activity during the expansion and maturation of the green fruit, more desirably continues active through the ripening or red fruit period. Comparable periods for other fruit are referred to as stages of ripening. The invention is not limited to those transcriptional initiation regions which are activated at or shortly after anthesis but also includes transcriptional initiation regions which are activated at any of the ripening stages of the fruit. The transcriptional initiation region may be native or homologous to the host or foreign or heterologous to the host. By foreign is intended that the transcriptional initiation region is not found in the wild-type host into which the transcriptional initiation region is introduced. Other fruit-specific promoters may be activated at times subsequent to anthesis, such as prior to or during the green fruit stage, during preripe (e.g., breaker) or even into the red fruit stage. Identifying useful transcriptional initiation regions may be achieved in a number of ways.

The promoter preferably comprises a transcriptional initiation regulatory region and translational initiation regulatory region of untranslated 5′ sequences, "ribosome binding sites", responsible for binding mRNA to ribosomes and translational initiation. The transcriptional initiation regulatory region may be composed of cis-acting subdomains which activate or repress transcription in response to binding of *trans* acting factors present in varying amounts in different cells. It is preferred that all of the transcriptional and translational functional elements of the initiation control region are derived from or obtainable from the same gene. In some embodiments, the promoter will be modified by the addition of sequences, such as enhancers, or deletions of non-essential and/or undesired sequences. By "obtainable" is intended a promoter having a DNA sequence sufficiently similar to that of a native promoter to provide for the desired specificity of transcription of a DNA sequence of interest. It includes natural and synthetic sequences as well as sequences which may be a combination of synthetic and natural sequences.

Of particular interest as a transcriptional initiation region is one which is activated at or shortly after anthesis, so that in the early development of the fruit, it provides the desired level of transcription of the sequence of interest. Normally, the expression product of the sequence of interest will be one which affects processes in the early formation of the fruit or which provides a property which is desirable during the growing (expansion) period of the fruit, or at or after harvesting.

To identify a promoter having the desired characteristics, where a fruit protein has been or is isolated, it may be partially sequenced, so that a probe may be designed for identifying messenger RNA specific for fruit. To further enhance the concentration of the messenger RNA specifically associated with fruit, cDNA may be prepared and the cDNA subtracted with messenger RNA or cDNA from non-fruit associated cells. The residual cDNA may then be used for probing the genome for complementary sequences, using an appropriate libray prepared from plant cells. Sequences which hybridize to the cDNA may then be isolated, manipulated, and the 5′-untranslated region associated with the coding region isolated and used in expression constructs to identify the transcriptional activity of the 5′-untranslated region. In some instances, a probe may be employed directly for screening a genomic library and identifying seqeunces which hybridize to the probe. The sequences will be manipulated as described above to identify the 5′-untranslated region.

A promoter from a tomato gene, referred to as 2All, was isolated using the methods described above. The 2All promoter provides for an abundant messenger, being activated at or shortly after anthesis and remaining active until the red fruit stage. Expression of the 2All gene under the 2All promoter occurs only in fruit; no expression is seen in root, leaves or stems. The gene encodes a sulfur-rich amino acid sequence similar to plant storage proteins in sulfur content and size.

cDNA clones made from ripe fruit were screened using cDNA probes made from ripe fruit, green fruit, and leaf mRNA. Clones were selected which has more intense hybridization with fruit DNAs as compared to the leaf cDNAs. The screening was repeated to identify a particular cDNA referred to as 2All. The 2All cDNA was then used for screening RNA from root, stem, leaf, and seven stages of fruit development after the mRNA was sized on gels. The screening demonstrated that the particular message was present throughout the seven stages of fruit development. The mRNA complementary to the specific cDNA was absent in other tissues which were tested. The cDNA was then used for screening a genomic library and a fragment selected which hybridized to the subject cDNA. The 5′ and 3′ non-coding regions were isolated and manipulated for insertion of a foreign sequence to be transcribed under the regulation of the 2All promoter. The degree of fruit-specificity of expression was then analyzed to identify initiation regions that would provide for at least the majority of expression of a DNA sequence of interest to be in fruit. Expression cassettes comprising a Gus reporter gene were prepared in which different amounts of the region 5′ to the 2All initiation start site were joined to the Gus gene.

Fruit-specific transcription suggests that gene regulatory proteins may be bound to enhancer sequences and other upstream promoter elements in fruit cells. By enhancer element ("enhancer") is intended a regulatory DNA sequence that is capable of activating transcription from a promoter linked to it with synthesis beginning at the normal RNA start site; which is capable of operating in both orientations (normal or flipped); and which is capable of functioning even when moved either upstream or downstream from the promoter. Both enhancers and other upstream promoter elements bind sequence specific DNA-binding proteins that mediate their effects.

To identify the exact nucleotide sequences important for the function of the enhancer(s), and other upstream elements, fragments of the 2All 5′-region are screened for their capacity to bind nuclear proteins and for their ability to function in a heterologous promoter. Binding experiments with nuclear proteins from fruit-tissue and other tissue such as leaf can be used to determine the presence of enhancer and silencer sequences; the protein binding studies can be used to pinpoint specific nucleotide sequences that bind to a corresponding series of gene regulatory proteins.

The activity of each enhancer and other upstream promoter elements generally is present on a segment of DNA which may contain binding sites for multiple proteins. The binding sites can generally be dissected by preparing smaller mutated versions of the enhancer sequence joined to a reporter gene whose product is easily measured, such as the Gus gene. The effect of each mutation on transcription can then be tested. Alternatively, fragments of this region can be prepared. Each of the mutated versions of the enhancer sequence or the fragments can be introduced into an appropriate host cell and the efficiency of expression of the reporter gene measured. Those nucleotides required for enhancer function in this test are then identified as binding sites for specific proteins by means of gel mobility shift and DNA foot printing studies.

An alternate means of examining the capability of the isolated fragments to enhance expression of the reporter gene is to look for sub-domains of the upstream region that are able to enhance expression levels from a promoter which comprises the TATACAAT box but shows little or no detectable activity. An example of such a promoter is the truncated 35S promoter (see for example Poulsen and Chua, *Mol. Gen. Genet.* - (1988) 214:16-23 and Benfey, et al., *EMBO J.* (1990) 9:1677-1684 and 1685-1696 and Gilmartin, *Plant Cell* - (1990) 2:269-378). A fragment of the 5′-region is inserted in front of the truncated promoter in an expression cassette, and the effect on expression of the reporter gene evaluated.

Of particular interest for fruit specific expression are regions capable of binding to nuclear proteins in the region up to about -3824 bp, particularly up to about -1825 bp from the mRNA start site of 2All. Within this region, several subdomains are of interest as having the characteristics of fruit specific enhancer elements. The enhancer elements are located within subdomains which include the region from about -737 to -104, particularly about -425 to -104; the region from about -1177 to -875; and the region from about -1805 to -1595. Also on interest an enhancer element located in a subdomain located between about -3824 and -1825 bp.

Other promoter regions of interest include those which regulate expression of the enzyme polygalacturonase, an enzyme which plays an important role in fruit ripening. The polygalacturonase promoter is active in at least the breaker through red fruit stage. In determining optimum amounts of other 5′ regions, such as that from the PG gene, which are required to give expression of a DNA sequence of interest preferentially in fruit, screening can be carried out as described above for the 2All 5′ region, using a reporter gene such as Gus.

A promoter from a gene expressed in fruit may be employed for varying the phenotype of the fruit. The transcription level should be sufficient to provide an amount of RNA capable of resulting in a modified fruit. By "modified fruit" is means fruit having a detectably different phenotype from a non-transformed plant of the same species, for example one not having the transcriptional cassette in question in its genome. Various changes in phenotype are of interest. These changes may include up- or down-regulation of formation of a particular saccharide, involving mono- or polysaccharides, involving such enzymes as polygalacturonase, levansucrase, dextransucrase, invertase, etc.; enhanced lycopene biosynthesis; cytokinin and monellin synthesis. Other properties of interest for modification include response to stress, organisms, herbicides, bruising, mechanical agitation, etc., change in growth regulators, organoleptic properties, etc. For antisense or complementary sequence transcription, the sequence will usually be at least 12, more usually at least 16 nt. Antisense sequences of interest include those of polygalacturonase, sucrase synthase and invertase.

The DNA sequence of interest may have any open reading frame encoding a peptide of interest, e.g., an enzyme, or a sequence complementary to a genomic sequence, where the genomic sequence may be an open reading frame, an intron, a non-coding leader sequence, or any other sequence where the complementary sequence will inhibit transcription, messenger RNA processing, e.g. splicing, or translation. The DNA sequence of interest may be synthetic, naturally derived, or combinations thereof. Depending upon the nature of the DNA sequence of interest, it may be desirable to synthesize the sequence with plant preferred codons. The plant preferred codons may be determined from the codons of highest frequency in the proteins expressed in the largest amount in the particular plant species of interest.

The termination region which is employed will be primarily one of convenience, since the termination regions appear to be relatively interchangeable. The termination region may be native with the transcriptional initiation region, may be native with the DNA sequence of interest, or may be derived from another source. Convenient termination regions are available from the Ti-plasmid of *A. tumefaciens*, such as the octopine synthase and nopaline synthase termination regions.

By appropriate manipulations, such as restriction, chewing back or filling in overhangs to provide blunt ends, ligation of linkers, or the like, complementary ends of the fragments can be provided for joining and ligation. In carrying out the various steps, cloning is employed, so as to amplify the amount of DNA and to allow for analyzing the DNA to ensure that the operations have occurred in proper manner. A wide variety of cloning vectors are available, where the cloning vector includes a replication system functional in *E. coli* and a marker which allows for selection of the transformed cells. Illustrative vectors include pBR332, pUC series, M13mp series, pACYC184, etc. Thus, the sequence may be inserted into the vector at an appropriate restriction site(s), the resulting plasmid used to transform the *E. coli* host, the *E. coli* grown in an appropriate nutrient medium and the cells harvested and lysed and the plasmid recovered. Analysis may involve sequence analysis, restriction analysis, electrophoresis, or the like. After each manipulation the DNA sequence to be used in the final construct may be restricted and jointed to the next seqeunce, where each of the partial constructs may be cloned in the same or different plasmids.

In addition to the transcription construct, depending upon the manner of introduction of the transcription construct into the plant, other DNA sequences may be required. For example, when using the Ti- or Ri-plasmid for transformation of plant cells, as described below, at least the right border and frequently both the right and left borders of the T-DNA of the Ti or Ri-plasmids will be joined as flanking regions to the transcription construct. The use of T-DNA for transformation of plant cells has received extensive study and is amply described in EPA Serial No. 120,516, Hoekema, In: The Binary Plant Vector System Offsetdrukkerij Kanters B.V., Alblasserdam, 1985, Chapter V, Knauf et al., *Genetic Analysis of Host Range Expression by Agrobacterium*, In: Molecular Genetics of the Bacteria Plant Interaction, Puhler, A. ed.,Springer-Verlag, NY, 1983, p.245, and An *et al., EMBO J.* (1985) 4:277-284.

Alternatively, to enhance integration into the plant genome, terminal repeats of transposons may be used as borders in conjunction with a transposase. In this situation, expression of the transposase should be inducible, so that once the transcription construct is integrated into the genome, it should be relatively stably integrated and avoid hopping.

The transcription construct will normally be joined to a marker for selection in plant cells. Conveniently, the marker may be resistance to a biocide, particularly an antibiotic, such a kanamycin, G418, bleomycin, hygromycin, chloramphenicol, or the like. The particular marker employed will be one which will allow for selection of transformed cells as compared to cells lacking the DNA which has been introduced.

A variety of techniques are available for the introduction of DNA into a plant cell host. These techniques include transformation with Ti-DNA employing *A. tumefaciens* or *A. rhizogenes* as the transforming agent, protoplast fusion, injection, electroporation, etc. For transformation with *Agrobacterium*, plasmids can be prepared in *E. coli* which plasmids contain DNA homologous with the Ti-plasmid, particularly T-DNA. The plasmid may or may not be capable of replication in *Agrobacterium*, that is, it may or may not have a broad spectrum prokaryotic replication system, e.g., RK290, depending in part upon whether the transcription construct is to be integrated into the Ti-plasmid or be retained on an independent plasmid. By means of a helper plasmid, the transcription construct may be transferred to the *A. tumefaciens* and the resulting transformed organism used for transforming plant cells.

Conveniently, explants may be cultivated with the *A. tumefaciens* or *A. rhizogenes* to allow for transfer of the transcription construct to the plant cells, the plant cells dispersed in an appropriate selective medium for selection, grown to callus, shoots grown and plantlets regenerated from the callus by growing in rooting medium. The *Agrobacterium* host will contain a plasmid having the *vir* genes necessary for transfer of the T-DNA to the plant cells and may or may not have T-DNA. For injection and electroporation, disarmed Ti-plasmids (lacking the tumor genes, particularly the T-DNA region) may be introduced into the plant cell.

As a host cell, any of a number of fruit bearing plants may be employed in which the plant parts of interest are derived from the ovary wall. These include true berries such as tomato, grape, blueberry, cranberry, currant, and eggplant; stone fruits (drupes) such as cherry, plum, apricot, peach, nectarine and avocado; compound fruits (druplets) such as raspberry and blackberry. In hesperidium (oranges, citrus), the expression cassette might be expected to be expressed in the "juicy" portion of the fruit. In pepos (such as watermelon, cantaloupe, honeydew, cucumber and squash) the equivalent tissue for expression is most likely the inner edible portions, whereas in legumes (such as peas, green beans, soybeans) the equivalent tissue is the seed pod.

The cells which have been transformed with an appropriate expression cassette may be grown into plants in accordance with conventional ways. See, for example, McCormick *et al., Plant Cell Reports* - (1986) 5:81-84. These plants may then be grown, and either pollinated with the same transformed strain or different strains, identifying the resulting hybrid having the desired phenotypic characteristic. Two or more generations may be grown to ensure that the subject phenotypic characteristic is stably maintained and inherited and then seeds harvested for use to provide fruits with the new phenotypic property.

The following examples are offered by way of illustration and not by limitation.

## EXPERIMENTAL

## Table of Contents

Example 1. Construction of Tomato Ripe Fruit CDNA Bank and Screening for Fruit-Specific Clones
Library Screening
Example 2. Analyses of cDNA Clones
Synthesis of RNA Probes
DNA Sequencing
Amino Acid Sequence
Southern Hybridization
Example 3. Preparation of Genomic Clone Plasmids
Isolation of a Genomic Clone
Preparation of pCGN1273
Preparation of pCGN1267
Example 4. Construction of Binary Vectors
Construction of pCGN783
(a) Construction of pCGN587
(b) Construction of pCGN739 Binary Vector
(c) Construction of pCGN726c (1 ATG-Kanamycin-3′ region)
(d) Construction of pCGN167
(e) Construction of pCGN766c (35S promoter-3′ region)
(f) Final Construction of pCGN783
Construction of Binary Vector pCGN1578
(a) Construction of pCGN1536
(b) Construction of pCGN1546
(c) Construction of pCGN565RB-H + X
(d) Construction of pCGN1542b
(e) Construction of pCGN1532
(f) Final Construction of pCGN1578
Example 5. Constructionof Tagged Genomic Cassettes and Binary Vectors
Isolation of Polygalacturonase Genomic Clone
Preparation of pCGN1268 and pCGN1269
Preparation of pCGN1219 and pCGN1220
Preparation of pCGN1255 and pCGN1258
Preparation of pCGN1227 and pCGN1228
Preparation of pCGN1264 and pCGN1265
Example 6. Preparationof Transgenic Plants
Example 7. Analysis of Tagged Genomic Constructs in Transgenic Plants
Northern Results on Transgenic Plants
Example 8. 2All Promoter Cassette
Transcriptional Initiation Region
Transcriptional and Translational
Termination Region
Final Construction
Construction of Plasmid pCGN1241

Construction of pCGN2610 and pCGN2611
Example 9. Comparison of Different Sized 2All 5′ Regions
Preparation of Test Constructs
Construction of pCGN2601 and pCGN2602
Construction of pCGN2812
Construction of pCGN2816
Construction of pCGN2813 and pCGN2814 (2All 5′-Gus-Tr5 3′ Constructs)
Analysis of Gus Enzyme Activity

E. coli strain pCGN1299x7118 was deposited with the American Type Culture Collection (ATCC), 12301 Parklawn Drive, Rockville, Maryland, 20852 on May 21, 1987 and given Accession No. 67408. Plasmid pCGN783 was deposited with the ATCC on December 23, 1988 and given Accession No. 67868. Plasmid pCGN1288 was deposited with the ATCC on July 13, 1989 and given Accession No. 68054.

## Example 1

## Construction of Tomato Ripe Fruit cDNA Bank and Screening for Fruit-Specific Clones

Tomato plants (*Lycopersicon esculentum* cv UC82B) were grown under greenhouse conditions. Poly-(A)$^+$ RNA was isolated as described by Mansson *et al., Mol. Gen. Genet.* (1985) 200:356-361. The synthesis of cDNA from poly(A)$^+$ RNA prepared from ripe fruit, cloning into the *Pst*I site of the plasmid pUC9 and transformation into and *E. coli* vector were all as described in Mansson *et al., Mol. Gen. Genet.* - (1985) *supra*.

## Library Screening

Two thousand recombinant clones were screened by colony hybridization with radiolabeled cDNA made from tomato red fruit mRNA, immature green fruit mRNA, and leaf mRNA. Bacterial colonies immobilized onto GeneScreen Plus filters (New England Nuclear), were denatured in 1.5 M NaCl in 0.5 M NaOH, then neutralized in 1.5 M NaCl in 0.5 M Tris-HCl pH 8, and allowed to air dry. Hybridization, washing and autoradiography were all performed as described in Maniatis *et al., Molecular Cloning: A Laboratory Manual* (1982) Cold Spring Harbor, New York.

Sixty-five clones were selected which had more intense hybridization signals with fruit cDNA than with leaf cDNA and therefore appeared to be under-represented in the leaf mRNA population relative to the fruit mRNA population. Replicate slot blot filters were prepared using purified DNA from the selected clones and hybridized with radioactive cDNA from leaf, green fruit, and red fruit as before. This allowed selection of cDNA clone 2All, also referred to as pCGN1299, which is on at high levels in both red and green fruit stages and off in the leaf.

## Example 2

## Analysis of cDNA Clones

## Synthesis of RNA Probes

The CDNA insert of pCGN1299 was excised as an *Eco*RI to *Hind*III fragment of approximately 600 bp (as measured on an agarose gel), and subcloned into the Riboprobe vector pGEMI (Pomega Biotec), creating pCGN488. $^{32}$P-labeled transcripts made from each strand of the pCGN488 insert using either SP6 or T7 polymerase were used as probes in separate Northern blots containing mRNA from leaf, immature green and mature red fruits. The RNA transcript from the SP6 promoter did not hybridize to the tomato mRNA. However, the transcript from the T7 promoter hybridized to an mRNA of approximatley 700 nt in length from the green fruit and the red fruit but not to mRNA from tomato leaf. The direction of transcription of the corresponding mRNA was thus determined.

The tissue specificity of the pCGN1299 cDNA was demonstrated as follows. RNA from root, stem, leaf, and seven stages of fruit development (immature green, mature green, breaker, turning, pink, light red, and red) was sized on formaldehyde/agarose gels according to the method described by Maniatis *et al.*, (1982) *supra*, immobilized on nitrocellulose and hybridized to $^{32}$P-labeled RNA which was synthesized *in vitro* from pCGN488 using T7 polymerase. Each lane contained 100 ng of polyA[+] RNA except for two lines (pink and light red lanes) which contained 10 $\mu$g of total RNA. Northern analysis of mRNA from root, stem, leaf, and fruit at various stages of development indicated that pCGN1299 cDNA was expressed in all stages of fruit development from the early stages immediately after anthesis to red ripe fruit. No MRNA hybridizing to pCGN1299 was found in leaf, stem, or root tissue. The size of the mRNA species hybridizing to the pCGN488 probe was approximatley 700 nt.

Message abundance corresponding to the pCGN1299 cDNA was determined by comparing the hybridization intensity of a known amount of RNA synthesized *in vitro* from pCGN488 using SP6 polymerase to mRNA from red tomato fruit in a Northern blot. The $^{32}$P-labeled transcript from pCGN488 synthesized *in vitro* using T7 polymerase was used as a probe. The Northern analysis was compared to standards which indicated that the pCGN1299 cDNA represents an abundant mRNA class in tomato fruit, being approximately 1% of the message.

DNA Sequencing

The polyA[+] sequence was missing from pCGN1299 cDNA. A longer cDNA clone, pCGN1298, therefore was identified by its hybridization with the pCGN488 probe. The complete DNA sequence of the two cDNA inserts was determined using both Maxam-Gilbert and the Sanger dideoxy techniques and is shown in Figure 1. The sequence of pCGN1298 contains additional seqeunces at both the 5′ and 3′ end compared to pCGN1299. As shown in Figure 1, the sequences are identical over the region that the two clones have in common.

Amino Acid Seqeunce

The pCGN1299 cDNA seqeunce was translated in three frames. The longest open reading frame (which starts from the first ATG) is indicated. Both pCGN1299 and pCGN1298 have an open reading frame which encodes a 96 amino acid polypeptide (see Figure 1). The protein has a hydrophobic N-terminus which may indicate a leader peptide for protein targeting. A hydrophobicity profile was calculated using the Hopp and Woods *(Proc. Natl. Acad. Sci. USA* (1981) 78:3824-3828) algorithm. Residues 10-23 have an extremely hydrophobic region.

A comparison of the 2All protein to pea storage proteins and other abundant storage proteins is shown in Figure 2. The sulfur-rich composition of the fruit-specific protein is similar to a pea storage protein which has recently been described (see Higgins, *et al., J. Biol. Chem.* (1986) 261:11124-11130, for references to the individual peptides). This may indicate a storage role for this abundant fruit-specific protein species.

Southern Hybridiazation

Southern analysis was performed as described by Maniatis *et al.*, (1982) *supra*. Total tomato DNA from cultivar UC82B was digested with *Eco*RI or *Hind*III, separated by agarose gel electrophoresis and transferred to nitrocellulose. Southern hybridization was performed using a $^{32}$P-labeled probe produced by nick translation of pCGN488 (Maniatis *et al.*, (1982) *supra*). The simple hybridization pattern indicated that the gene encoding pCGN1299 cDNA was present only in a few copies or perhaps even only one copy in the tomato genome.

Example 3

## Preparation of Genomic Clone Plasmids

Two genomic clone plasmids were prepared, pCGN1273 and pCGN1267. They were obtained as follows.

### Isolation of a Genomic Clone

A genomic library established in Charon35/*Sau*3A constructed from DNA of the tomato cultivar VFNT-Cherry was screened using the [32PI-RNA from cDNA clone pCGN488 as a probe. A genomic clone containing approximately 12.5 kb of sequence from the tomato genome was isolated. The region which hybridizes to a pCGN488 probe spans an *Xba*I restriction site which was found in the cDNA sequence and includes the transcriptional initiation region designated 2AII.

### Preparation of pCGN1273

The region surrounding the *Xba*I restriction site, approximately 2.4 kb in the 5′ direction and approximately 2.1 kb in the 3′ direction was subcloned to provide an expression cassette. The 5′ *Xho*I to *Xba*I fragment and the 3′ *Xba*I to *Eco*RI fragment from the 2AII genomic clone were inserted into a pUC-derived chloramphenicol plasmid containing a unique *Xho*I site and no *Xba*I site. This promoter cassette plasmid is called pCGN1273. The complete sequence of the 2AII genomic DNA cloned into pCGN1273 from the *Xho*I site (position 1 at the 5′ end) to the *Eco*RI site (position 4654) was determined by Sanger dideoxy techniques and is shown in Figure 3. The sequence of the genomic clone is identical to the pCGN1299 cDNA clone over the region they have in common. The seqeunce reported previously (see USSN 188,361 filed April 29, 1988) corresponds to position 1169 to 2645 of the complete sequence.

### Preparation of pCGN1267

pCGN1267 was constructed by deleting from pCGN1273 a portion of the plasmid poly-linker from the *Eco*RV site to the *Bam*HI site. Two DNA seqeunces were inserted into pCGN1273 at the unique *Xba*I site (position 2494). This site is in the 3′ non-coding region of the 2AII genomic clone before the poly A site.

## Example 4

## Construction of Binary Vector

### Construction of pCGN783

pCGN783 is a binary plasmid containing the left and right T-DNA borders of *A. tumefaciens* octopine Ti-plasmid pTiA6 (Currier and Nester, *J. Bacteriol.* (1976) 126:157-165) the gentamicin resistance gene of pPHIJI (Hirsch *et al., Plasmid* (1984) 12:139-141), the 35S promoter of cauliflower mosaic virus (CaMV) Gardner *et al., Nucleic Acid Res.* (1981) 9:1871-1880); the kanamycin resistance gene of Tn5 (Jorgensen, *Mol. Gen.* (1979) 177:65); and the 3′ region from transcript 7 of pTiA6 (Currier and Nester, *supra* (1976)). A schematic diagram of the construction of pCGN783 is shown in Figure 4. (a) through (f) refer to the plasmid constructions detailed below.

### (a) Construction of pCGN587

The *Hind*III-*Sma*I fragment of Tn5 containing the entire structural gene for APH3′II (Jorgensen *et al.,*

*Mol. Gen.* 1979) 177:65), was cloned into pUC8 (Vieira and Messing, *Gene* (1982) 19:259), converting the fragment into a *Hind*III-*Eco*RI fragment, since there is an *Eco*RI site immediately adjacent to the *Sma*I site. The *Pst*I-*Eco*RI fragment, pCGN300, containing the 3' portion of the APH3'II gene, was then combined with an *Eco*RI-*Bam*HI-*Sal*I-*Pst*I linker into the *Eco*RI site of pUC7 (pCGN546W). Since this construct does not confer kanamycin resistance, kanamycin resistance was obtained by inserting the *Bgl*I-*Pst*I fragment of the APH3'II gene into the *Bam*HI-*Pst*I site (pCGN546X). This procedure reassembles the APH3'II gene, so that *Eco*RI sites flank the APH3'II gene. An ATG codon was upstream from and out of reading frame with the ATG initiation codon of APH3'II. The undesired ATG was avoided by inserting a *Sau*3A-*Pst*I fragment from the 5' end of APH3'II, which fragment lacks the superfluous ATG, into the *Bam*HI-*Pst*I site of pCGN546W to provide plasmid pCGN550. The *Eco*RI fragment of pCGN550 containing the APH3'II gene was then cloned into the *Eco*RI site of pUC8-PUC13 (K. Buckley, Ph.D. Thesis, University of California, San Diego, 1985) to give pCGN551.

Each of the *Eco*RI fragments containing the APH3'II gene was then cloned into the unique *Eco*RI site of pCGN451, which contains an octopine synthase cassette for expression to provide pCGN548 (2ATG) and pCGN552 (1ATG). The plasmid pCGN451 having the ocs 5' and the ocs 3' in the proper orientation was digested with *Eco*RI and the *Eco*RI fragment from pCGN551 containing the intact kanamycin resistance gene inserted with *Eco*RI site to provide pCGN552 having the kanamycin resistance gene in the proper orientation. This ocs/KAN gene was used to provide a selectable marker for the trans type binary vector pCGN587.

The 5' portion of the engineered octopine synthase promoter cassette consists of pTiA6 DNA from the *Xho*I at bp 15208-13644 (Barker *al al., supra* (1983)), which also contains the T-DNA boundary sequence (border) implicated in T-DNA transfer. In the plasmid pCGN587, the osc/KAN gene from pCGN552 provides a selectable marker as well as the right border. The left boundary region was first cloned in M13mp9 as a *Hind*III-*Sma*I piece (pCGN502) (base pairs 602-2212) and recloned as a *Kpn*I-*Eco*RI fragment in pCGN565 to provide pCGN580. pCGN565 is a cloning vector based on pUC8-Cm, but containing pUC18 linkers. pCGN580 was linearized with *Bam*HI and used to replace the smaller *Bgl*I fragment of pVCK102 (Knauf and Nester, *Plasmid* (1982) 8:45), creating pCGN585. By replacing the smaller *Sal*I fragment of pCGN585 with the *Xho*I fragment from pCGN552 containing the ocs/KAN gene, pCGN587 was obtained.

## (b) Construction of pCGN739 (Binary Vector)

To obtain the gentamicin resistance marker, the resistance gene was isolated from a 3.1 kb *Eco*RI-*Pst*I fragment of pPHIJI (Hirsch *et al., Plasmid* (1984) 12:139-141) and cloned into pUC9 (Vieira *et al., Gene* - (1982) 19:259-268) yielding pCGN549. The pCGN549 *Hind*III-*Bam*HI fragment containing the gentamicin resistance gene replaced the *Hind*III-*Bgl*II fragment of pCGN587 (for construction, see Example 4(a) above) creating pCGN594.

The pCGN594 *Hind*III-*Bam*HI region which contains an *ocs*-kanamycin-*ocs* fragment was replaced with the *Hind*III-*Bam*HI poly-linker region from pUC18 (Yanisch-Perron, *Gene* (1985) 33:103-119) to make pCGN739.

## (c) Construction of 726c (1 ATG-Kanamycin-3' region)

pCGN566 contains the *Eco*RI-*Hind*III linker of pUC18 (Yanisch-Perron, *ibid.*) inserted into the *Eco*RI-*Hind*III sites of pUC13-Cm (K. Buckley, (1985) *supra*). The *Hind*III-*Bgl*II fragment of pNW31c-8, 29-1 (Thomashow *et al., Cell* (1980) 19:729) containing ORF1 and 2 (Barker *et al., Plant Mol. Biol.* (1984) 2:335-350) was subcloned into the *Hind*III-*Bam*HI sites of pCGN566 producing pCGN703.

The *Sau*3A fragment of pCGN703 containing the 3' region of transcript 7 from pTiA6 (corresponding to bases 2396-2920 of pTi15955 (Barker *et al., supra* (1984)) was subcloned into the *Bam*HI site of pUC18 (Yanisch-Perron *et al., supra* (1985)) producing pCGN709.

The *Eco*RI-*Sma*I poly-linker region of pCGN709 was replaced with the *Eco*RI-*Sma*I fragment from pCGN587 (see Example 4(a), above) which contains the kanamycin resistance gene (APH3'II) producing pCGN726.

The *Eco*RI-*Sal*I fragment of pCGN726 plus the *Bgl*II-*Eco*RI fragment of pCGN734 were inserted into the *Bam*HI-*Sal*I sites of pUC8-pUC13-cm (Buckley, (1985), supra ) producing pCGN738. To construct pCGN734, the *Hind*III-*Sph*I site of M13mp19 (Norrander *et al., Gene* (1983) 26:101-106). Using an oligonucleotide corresponding to bases 3287 to 3300, DNA synthesis was primed from this template.

Following SI nuclease treatment and *Hind*III digestion, the resulting fragment was cloned into the *Hind*III-*Sma*I site of pUC19 (Yanisch-Perron *et al., supra* (1985)). The resulting *Eco*RI to *Hind*III fragment of pTiA6 (corresponding to bases 3390-4494) was inserted into the *Eco*RI site of pUC8 (Vieira and Messing, *supra* - (1982)) resulting in pCGN734. pCGN726c is derived from pCGN738 by deleting the 900 bp *Eco*RI-*Eco*RI fragment.

### (d) Construction of pCGN167

pCGN167 is a construct containing a full length CaMV promoter, 1 ATG-kanmycin gene, 3' end and the bacterial Tn903-type kanamycin gene. MI is an *Eco*RI fragment from pCGN5550 (see construction of pCGN587) and was cloned into the *Eco*RI cloning site in the 1 ATG-kanamycin gene proximal to the poly-linker region of M13mp9. See copending Application Serial No. 920,574, filed October 17, 1986, which disclosure is incorporated herein by reference.

To construct pCGN167, the *Alu*I fragment of CaMV (bp 7144-7735) (Gardner *et al., Nucl. Acids Res.* - (1981) 9:2871-2888) was obtained by digestion with *Alu*I and cloned into the *Hinc*II site of M13mp7 (Vieira, *Gene* (1982) 19:259) to create C614. an *Eco*RI digest of C614 produced the *Eco*RI fragment from C614 containing the 35S promoter which was cloned into the *Eco*RI site of pUC8 (Vieira *et al., Gene* (1982) 19:259) to produce pCGN146. To trim the promoter region, the *Bgl*I site (bp 7670) was treated with *Bgl*I and Bal31 and subsequently a *Bgl*I linker was attached to the Bal31 treated DNA to produce pCGN147.

pCGN148a containing the promoter region, selectable marker (KAN with 2 ATGs) and 3' region was prepared by digesting pCGN528 with *Bgl*I and inserting teh *Bam*HI-*Bgl*I promoter fragment from pCGN147. This fragment was cloned into the *Bgl*I site of pCGN528 so that the *Bgl*I site was proximal to the kanamycin gene of pCGN528. pCGN528 was made as follows. pCGN525 was made by digesting a plasmid containing Tn5 which harbors a kanamycin gene (Jorgenson *et al., Mol. Gen.* (1979) 177:65) with *Hind*III-*Bam*HI and inserting the *Hind*III-*Bam*HI fragment containing the kanamycin gene into the *Hind*III-*Bam*HI sites in the tetracycline gene of pACYC184 (Chang and Cohen, *J. Bacteriol.* (1978) 134:1141-1156). pCGN526 was made by inserting the *Bam*HI fragment 19 of pTiA6 (Thomashow *et al., Cell* (1980) 19:729-739( into the *Bam*HI site of pCGN525. pCGN528 was obtained by deleting the small *Xho*I fragment from pCGN526 by digesting with *Xho*I and relegating.

pCGN149a was made by cloning the *Bam*HI kanamycin gene fragment from pMB9KanXXI into the *Bam*HI site of pCGN148a. pMB9KanXXI is a pUC4K variant (Vieira and Messing, *Gene* (1982) 19:259-268) which has the *Xho*I site missing but contains a functional kanamycin gene from Tn903 to allow for efficient selection in *Agrobacterium*. pCGN149a was digested with *Bgl*I and *SPh*I. This small *Bgl*I-Sphl fragment o pCGN149a was replaced with the *Bam*HI-Sphl fragment from MI (see blow) isolated by digestion with *Bam*HI and *SPh*I. This produces pCGN167.

### (e) Construction of pCGN766c (35S promoter-3' region)

The *Hind*III-*Bam*HI fragment of pCGN167 containing the CaMV-35S promoter, 1 ATG-kanamycin gene and the *Bam*HI fragment 19 of pTiA6 was cloned into the *Bam*HI-*Hind*III sites of puC19 (Norrander *et al.,* (1985), *supra*; Yanisch-Perron *et al.,* (1985), *supra*) creating pCGN976.

The 35S promoter and 3' region from transcript 7 was developed by inserting a 0.7 b *Hind*III-*Eco*RI fragment of pCGN976 (35S promoter) and the 0.5 kb *Eco*RI-*Sal*I fragment of pCGN709 (transcript 7:3'; for construction see *supra*) into the *Hind*III-*Sal*I sites of pCGN566 creating pCGN766c.

### (f) Final Construction of pCGN783

The 0.7 kb *Hind*III-*Eco*Ri fragment of pCGN766c (CaMV-35S promoter) was ligated to the 1.5 kb *Eco*RI*Sal*I fragment of pCGN726c (1-ATG-KAN-3' region) into the *Hind*III-*Sal*I sites of pUC119 (J. Vieira, Rutgers University, New Jersey) to produce pCGN778. The 2.2 kb region of pCGN778, a *Hind*III-*Sal*I fragment containing the CaMV35S promoter (1-ATG-KAN-3' region), replaced the *Hind*III-*Sal*I poly-linker region of pCGN739 to produce pCGN783.

A DNA construct comprising a DNA sequence of interest may be inserted into the binary plasmid containing a plant kanamycin resistance marker, between the left and right borders. The resulting plasmid binary vector, in a host microorganism such as *E. coli* C2110, is conjugated into *A. tumefaciens* containing a disarmed Ti-plasmid capable of transferring the DNA sequence of interest and the kanamycin resistance cassette into the plant host genome. The *Agrobacterium* system which is employed is *A. tumefaciens* PC2760 (G. Ooms *et al., Plasmid* (1982) 7:15-29; Hoekema *et al., Nature* (1983) 303:179-181; European Patent Application 84-200239.6, 2424183, which disclosures are incorporated herein by reference.

Construction of Binary vector pCGN1578

pCGN1578 is a binary plant transformation vector containing the left and right T-DNA borders of *Agrobacterium tumefaciens* octopine Ti-plasmid pTiA6 (Currier and Nester, *J. Bact.* (1976) 126:157-165), the gentamicin resistance gene of pPHIJI (Hirsch and Beringer, *Plasmid* (1984) 12:139-141), an *Agrobacterium rhizogenes* Ri plasmid origin of replication from pLJbB11 (Jouanin *et al., Mol. Gen. Genet.* (1985) 01:370-374), a 35S promoter-Kan$^R$-tml 3' region capable of conferring kanamycin resistance to transformed plants, a ColEI origin of replication from pBR322 (Bolivar *et al., Gene* (1977) 2:95-113), and a *lacZ'* screenable marker gene from pUC18 (Yanisch-Perron *et al., Gene* (1985) 53:103-119).

(a) Construction pCGN1536

A 54. kb *Eco*RI fragment was removed from pVK2323 (Knauf and Nester, *Plasmid* (1982) 8:45), by *Eco*RI digestion and cloned into *Eco*RI digested pACYC184 (Chang and Cohen, *J. Bacteriol.* (1978) 134:1141-1156) to create pCGN14. The 1434 bp *Cla*I-*Sph*I fragment of pCGN14, containing the mas 5' region (bp 20128-21562) according to numbering of Barker *et al., Plant Mol.* Biol. (1983) 2 :335-350 was cloned into *Acc*I-*Sph*I digested pUC19 (Yanisch-Perron *et al., Gene* (1985) 53:103-119) to generate pCGN40. A 746 bp *Eco*RV-*Nae*I fragment of the mas 5' region was replaced by any *Xho*I site by digesting pCGN40 with *Eco*RV and *Nae*I followed by ligation in the presence of synthetic *Xho*I linker DNA to create pCGN1036. The 765 bp *Sst*I-*Hind*III fragment (bp 18474-1929) of pCGN14, containing the mas 3' region, was cloned into *Sst*I-*Hind*III digester pUC18 (Norrander *et al., Gene* (1983) 26:101-106) to yield pCGN43. The *Hind*III site of pCGN43 was replaced with an *Eco*RI site by digestion with *Hind*III, blunt ending with Klenow Enzyme, and ligation with synthetic *Eco*RI linker DNA to create pCGN1034. The 767 bp *Eco*RI fragment of pCGN1034 was cloned into *Eco*RI-digested pCGN1036 in the orientation that places bp 19239 of the mas 3' region proximal to the mas 5' region to create pCGN1040. pCGN1040 was subjected to partial digestion with *Sst*I, treated with T4 DNA polymerase to create blunt ends, and ligated in the presence of synthetic *Xho*I linker DNA. A clone was selected in which only the *Sst*I site at the junction of bp 18,474 (mas DNA) and the vector DNA was replaced by an *Xho*I site to generate pCGN1047.

pCGN565 (a cloning vector based upon pUC8-cm but containing pUC18 linkers) was digested with *Eco*RI and *Hind*III, treated with Klenow enzyme to create blunt ends, and ligated in the presence of synthetic *Xho*I linker DNA to create pCGN1003; this recreated with *Eco*RI site adjacent to the *Xho*I linker. pCGN1003 was digested with *Eco*RI, treated with Klenow enzyme to create blunt ends, and ligated in the presence of synthetic *Pst*I linker DNA to create pCGN1007. The 1.5 kg *Xho*I fragment of pCGN1047, containing the mas 5' region and the mas 3' region with a multiple cloning site between, was cloned into *Xho*I digested pCGN1007 to construct pCGN1052. A portion of the multiple cloning site of pCGN1052 was deleted by digestion with *Xba*I and *Sst*I, treated with Klenow enzyme to make blunt ends, and ligated to generate pCGN1052ΔXS.

To prepare pCGN50, the *Hind*III-*Sma*I fragment of Tn5 containing the entire structural gene for APHII (Jorgensen, *et al., Mol. Gen. Genet.* (1979) 177:65) was cloned into pUC8 (Vieira and Messing, *Gene* - (1982) 19 :259), converting the fragment into a *Hind*III-*Eco*RI fragment, since there is an *Eco*RI site immediately adjacent to the *Sma*I site. The *Pst*I-*Eco*RI fragment of pCGN300, containing the 3' portion of the *APH*II gene, was then combined with an *Eco*RI-*Bam*HI-*Sal*I-*Pst*I linker into the *Eco*RI site of pUC7 (pCGN546W). Since this construct does not confer kanamycin resistance, kanamycin resistance was obtained by inserting the *Bgl*II-*Pst*I fragment of the *APH*II gene into the *Bam*HI-*Pst*I site (pCGN546X). This procedure reassembles the *APH*II gene, so that *Eco*RI sites flank the gene. An ATG codon was upstream from and out of reading frame with the ATG initiation codon of *APH*II. The undesired ATG was avoided by inserting a *Sau*3A-*Pst*I fragment from the 5' end of *APH*II, which fragment lacks the superfluous ATG, into the *Bam*HI-*Pst*I site of pCGN546W to provide plasmid pCGN550.

The 1 kb *Eco*RI-*Sma*I fragment of pCGN550 containing the 1 ATG-kanamycin resistance gene, was cloned into *Eco*RI-*Sma*I digested to create pBSKm; this plasmid contained an M13 region allowing generation of single stranded DNA. Single stranded DNA was generated according to the supplier's recommendations, and *in vitro* mutagenesis was performed (Adelman *et al.,* DNA (1983) 2:183-193) using a synthetic oligonucleotide with the seqeunce 5′GAACTCCAGGACGAGGC3′ to alter a *Pst*I site within the kanamycin resistance gene, creating pCGN1534. pCGN1534 was digested with *Sma*I and ligated in the presence of a synthetic *Eco*RI linker DNA to generate pCGN1535. The 1 kb *Eco*RI fragment of pCGN1535 was cloned into *Eco*RI digested pCGN1052ΔXS to create the mas 5′-kan-mas 3′ plant selectable marker cassette pCGN1536.

(b) Construction of pCGN1546

pCGN149a (see above) was digested with *Hin*dIII and *Bam*HI and ligated to pUC8 digested with *Hin*dIII and *Bam*HI to produce pCGN169. This removed the Tn903 kanamycin marker. pCGN565 (a cloning vector based on pUC8-Cm but containing pUC18 linkers) and pCGN169 were both digested with *Hin*dIII and *Pst*I and ligated to form pCGN203, a plasmid containing the CaMV 35S promoter and part of the 5′-end of the Tn5 kanamycin gene (up to the *Pst*I site, (Jorgenson *et al.,* (1979), *supra*). A 3′-regulatory region was added to pCGN203 from pCGN204. pCGN204 was made by cloning the *Eco*RI fragment of CaMV (bp 408-6105) containing the region V 3′ region into pUC18 (Gardner, *et al.* (1981) *supra*). pCGN203 and pCGN204 were digested with *Hin*dIII and *Pst*I and the 3′ regulatory region of pCGN204 was inserted into pCGN203. The resulting cassette, pCGN206, was digested with *Hin*dIII. The ends were filled-in with Klenow polymerase and *Xho*I linkers were added. The resulting plasmid was called pCGN986X. pBRX25 (construction of this plasmid is described in European application EPA 0 229 042 filed January 7, 1987, which application is incorporated herein by reference) contains only 11 bp of the 5′ untranslated region of the nitrilase gene. The *Bam*HI-*Sac*I fragment of pBRX25 was inserted into *Bam*HI-*Sac*I digested pCGN986X yielding pBRX66. pBRX68 was digested with *Pst*I and *Eco*I, blunt ends generated by treatment with Klenow polymerase, and *Xho*I linkers added. The resulting plasmid, pBRX68, had a *tml* 3′ region of approximately 1.1 kb. pBRX68 was digested with *Sal*I and *Sac*I, blunt ends generated by treatment with Klenow polymerase, and *Eco*RI linkers added. The resulting plasmid, pCGN986XE, is a 35S promoter-tml 3′ expression cassette lacking the nitrilase gene. The Tn5 kanamycin resistance gene was then inserted into pCGN986XE. The 1.0 kb *Eco*RI fragment of pCGN1536 was ligated into pCGN986XE digested with *Eco*RI. A clone with the Tn5 kanamycin resistance gene in the correct orientation for transcription and translation was chosen and called pCGN1537b. The 35S promoter Kan[R]-tml 3′ region was then transferred to a chloramphenicol resistant plasmid backbone, pCGN786. pCGN786 is a pUC-CAM based vector with the synthetic oligonucleotide 5′ GGAATTCGTCGACAGATCTCTGCAGCTCGAGGGATCCAAGCTT 3′ containing the cloning sites *Eco*RI, *Sal*I, *Bgl*II, *Pst*I, *Xho*I, *Bam*HI, and *Hin*dIII inserted into pCGN566. pCGN566 contains the *Eco*RI-*Hin*dIII linker of pUC18 inserted into the *Eco*RI-*Hin*dIII sites of pUC13-Cm. pCGN786 was digested with *Xho*I and the *Xho*I fragment of pCGN1537b containing the 35S promoter-Kan[R]-tml 3′ region was ligated in. The resulting clone was termed pCGN1546.

(c) Construction of pCGN565RB-H + X

pCGN451, which includes an octopine cassette containing about 1556 bp of the 5′ non-coding region of the octopine synthase gene fused via an *Eco*RI linker to the 3′ non-coding region of the octopine synthase gene of pTiA6, was digested with *Hpa*I and ligated in the presence of synthetic *Sph*I linker DNA to generate pCGN55. The pTi coordinates are 11,207 to 12,823 for the 3′ region and 13,643 to 15,208 for the 5′ region as defined by Barker *et al.,* Plant Mol. Biol. (1983) 2:325. The *Xho*I-*Sph*I fragment of pCGN55 (bp 13,800-15208, including the right border of *Agrobacterium tumefaciens* T-DNA; (Barker *et al.,* Gene (1977) 2:95-113) was cloned into *Sal*I-*Sph*I digested pUC19 (Yanisch-Perron *et al.,* Gene (1985) 53:103-119) to create pCGN60. The 1.4 kb *Hin*dIII-*Bam*HI fragment of pCGN60 was cloned into *Hin*dIII-*Bam*HI digested pSP64 (Promega, Inc.) to generate pCGN1039. pCGN1039 was digested with *Sma*I and *Nru*I (deleting bp 14,273-15,208; (Barker *et al.,* Gene 1977) 2:95-113) and ligated in the presence of synthetic *Bgl*II linker DNA creating pCGN1039ΔNS. The 0.47 kb *Eco*RI-*Hin*dIII fragment of pCGN1039ΔNS was cloned into *Eco*RI-*Hin*dIII digested pCGN565 to create pCGN565RB. pCGN565 is a cloning vector based on pUC8Cm but containing pUC18 linkers. The *Hin*dIII site of pCGN565RB was replaced with an *Xho*I site by *Hin*dIII digestion, treatment with Klenow enzyme, and ligation in the presence of synthetic *Xho*I linker DNA to create pCCN565RB-H + X.

(d) Construction of pCGN1542b

pCGN65 was constructed as follows. First, pCGN501 was constructed by cloning a 1.85 kb EcoRI-XhoI fragment of pTiA6 (Currier and Nester, *J. Bact.* (1976) 126:157-165) containing bases 13,362-15,208 (Barker, *et al., Plant Molec. Biol.* (1983) 2:335-350) of the T-DNA (right border), into EcoRI-SaII digested M13mp9 (Vieira and Messing, *Gene* (1982) 19:259-268). pCGN502 was constructed by cloning a 1.6 kb HindIII-SmaI fragment of pTiA6, containing bases of 602-2,212 of the T-DNA (left border), into HindIII-SmaI digested M13mp9. pCGN501 and pCGN502 were both digested with EcoRI and HindIII and both T-DNA-containing fragments cloned together into HindIII digested pUC9 (Vieira and Messing, *Gene* (1982) 19:259-268) to yield pCGN503, containing both T-DNA border fragments. pCGN503 was digested with HindIII and EcoRI and the two resulting HindIII-EcoRI fragments (containing the T-DNA borders) were cloned into EcoRI digested pHC79 (Hohn and Collin, *Gene* (1980) 11:291-298) to generate pCGN518. The KpnI-EcoRI fragment from pCGN518, containing the left T-DNA border, was cloned into KpnI-EcoRI digested pC1GN565 to generate pCGN580. The BamHI-BgIII fragment of pCGN580 was cloned into the BamHI site of pACYC184 (Chang and Cohen, *J. Bacteriol.* (1978) 134:1141-1156) to create pCGN51. The 1.4 kb BamHI-SphI fragment of pCGN60 (see pCGN65x2X section above) containing the T-DNA right border fragment, was cloned into BamHI-SphI digested pCGN51 to create pCGN65.

To make pCGN65ΔKX-S + X, pCGN65 was digested with KpnI and XbaI, treated with Klenow enzyme to create blunt ends, and ligated in the presence of synthetic BqlII linker DNA.

pUC18 was digested with HaeII to release the lacZ′ fragment, treated with Klenow enzyme to create blunt ends, and the lacZ-containing fragment ligated into pCGN565RB-H + X, which had been digested with AccI and SphI and treated with Klenow enzyme, resulting in pCGN565RBα3X. In pCGN565RBα3X, the lac promoter is distal to the right border. Both clones were positive for lacZ′ expression when plated on an appropriate host. Each contain bp 13990-142773 of the right border fragment (Barker *et al.* (1983) *supra*), the AccI-SphI fragment (bp 13800-13990) having been deleted. The 728 bp BgIII-Xho I fragment of pCGN565RBα3X, containing the T-DNA right border piece and the lacZ′ gene, were cloned into BgIII-XhoI digested pCGN65ΔKX-S + X replacing the BgIII-XhoI right border fragment of pCGN650ΔKX-S +, to create pCGN65α3X.

The ClaI fragment from pCGN65α3X was deleted and replaced with an XhoI linker by digesting with ClaI, treating with Klenow enzyme to crate blunt ends, and ligating in the presence of synthetic XhoI linker DNA to create pCGN65α3XX. pCGN65α3XX was digested with BgIII and EcoRV, treated with Klenow polymerase, and BgIII linkers added. The resulting plasmid, pCGN65α3XX′, lacked an approximately 20 bp piece of DNA that was present in pCGN65α3XX.

pBR322 (Boliver *et al., Gene* (1977) 2:95-113) was digested with EcoRI and PvuII, treated with Klenow polymerase to generate blunt ends, and BgIII linkers added. An ampicillin resistant, tetracycline sensitive clone, pCGN1538, was selected. This clone lacked the approximately 2.2 kb EcoRI-PvuII fragment containing the tetracycline resistance gene. The PvuII site was lost but the EcoRI site was regenerated upon addition of BgIII linkers.

pCGN65α3XX′ was digested with BgIII and ligated to BgIII digested pCGN1538 to create pCGN1542a which contained both plasmid backbones. pCGN1542a was digested with XhoI and relegated. An ampicillin resistant, chloramphenicol sensitive clone was chosen which lacked the pACYC184 backbone, creating pCGN1542b.

(e) Construction of pCGN1532

The 3.Sb EcoRI-PstI fragment containing the gentamicin resistance gene was removed from pPHIJI (Hirsch and Beringer, *Plasmid* (1984) 12:139-141) by EcoRI-PstI digestion and cloned into EcoRI-PstI digested pUC9 (Vieira and Messing, *Gene* (1982) 19:259-268) to generate pCGN549. HindIII-PstI digestion of pCGN549 yielded a 3.1 kb fragment bearing the gentamicin resistance gene, which was made blunt ended by the Klenow fragment of DNA polymerase I and cloned into PvuII digested pBR322 (Bolivar *et al., Gene* (1977) 2:95-113) to create pBR322Gm. pBR322Gm was digested with DraI and SphI, treated with Klenow enzyme to create blunt ends, and the 2.8 kb fragment cloned into the Ri origin containing plasmid pLJbB11 (Jouanin *et al., Mol. Gen. Genet.* (1985) 201:370-374) which has been digested with ApaI and made blunt ended with Klenow enzyme, creating pLHbB11Gm. The extra ColEI origin and the kanamycin resistance gene were deleted from pLJBIIGm by digestion with BamHI, followed by self-closure to create pBMBII. The HindIII site of pGmBII was deleted by HindIII digestion, followed by treatment with Klenow enzyme and self-closure, creating pGMBII-H. The PstI site of pGmGII-H was deleted by PstI digestion,

followed by treatment with Klenow enzyme and self-closure, creating pCGN1532.

(f) Final Construction of pCGN1578

The *XhoI* fragment of pCGN1548 containing the 35S promoter-Kan$^R$-tml 3$'$ region was cloned into *XhoI* digested 1542b to create pCGN1556. The *XhoI* fragment from pCGN1546 was oriented within 1542b such that the order of components was: *left border*-35S promoter-Kan$^R$-tml 3$'$-*lacZ$'$-right border*.

The T-DNA containing *BglI* fragment of pCGN1556 was cloned into *BamHI* restricted pCGN1532 resulting in the binary vector, pCGN1578. In pCGN1578, the orientation of the insert was such that the T-DNA left border was adjacent to the Ri plasmid origin of replication. This binary vector has several advantages, including a minimal amount of DNA between the T-DNA borders, high stability in *Agrobacterium* hosts, high copy number in *E. coli* hosts and blue/white screen with multiple restriction enzyme sites for ease of cloning target DNA.

Example 5

Construction of Tagged Genomic Cassettes and Binary Vectors

The 2All genomic fragment was tagged with PG cDNA sense or antisense sequences, PG genomic DNA antisense sequence or DMA transferase (tmr) genomic DNA sequences in antisense or sense orientations. PG DNA sequences were inserted into the unique *XbaI* site of the pCGN1273 or the unique *ClaI* site of the pCGN1267 promoter cassettes. *tmr* seqeunces were inserted into the unique *ClaI* site of pCGN1261. A summary of the tagged genomic cassettes prepared is shown in Table 1 below. The inserted seqeunces would increase the size of the mRNA over the endogenous transcription, and thus the expression pattern of the construct could be compared to the endogenous gene by a single Northern hybridization in a manner analogous to the detection of a tuber-specific potato gene described by Eckes *et al., Mol. Gen. Genet.* (1986) 205:14-22.

Table 1

| Summary of Tagged Cassettes Constructed | | | | |
|---|---|---|---|---|
| Genomic Clone Plasmid | "Tagged" Plasmid | Insertion | Orientation | Binary |
| pCGN1273 | pCGN1270 | PGcDNA | Sense | pCGN1268 |
| | pCGN1271 | PGcDNA | Antisense | pCGN1269 |
| | pCGN1215 | PG Genomic DNA | Antisense | pCGN1219 & pCGN1220 |
| pCGN1267 | pCGN1263 | PGcDNA | Sense | pCGN1260 |
| | pCGN1262 | PGcDNA | Antisense | pCGN1255 & pCGN1258 |
| | pCGN1225 | PG Genomic DNA | Antisense | pCGN1227 & pCGN1228 |
| | pCGN1266 | tmr Genomic DNA | Sense | pCGN1264 & pCGN1265 |

Isolation of Polygalacturonase Genomic Clone

An *EcoRI* partial genomic library established in Charon 4 constructed from DNA of a *Lycopersicon esculentum* cultivar was screened using a probe from polygalacturonase (PG) cDNA (Sheehy *et al., Mol. Gen. Genet.* (1987) 208:30-36). A lambda clone containing an approximately 16 kb insert as isolated from the library. An internal 2207 bp *HindIII* to *EcoRI* was sequenced. The *HindIII-EcoRI* fragment includes the

PG promoter region.

Sequence of Genomic Clone

The DNA sequence of the genomic clone was determined by Sanger dideoxy techniques and is as shown in Figure 5. The sequence of the genomic clone bases 1427 to 1748 are homologous to the polygalacturonase cDNA sequence.

Preparation of pCGN1268 and pCGN1269

pCGN1273 was tagged with 383 bp (from base number -23 in the polylinker region to 360) from the 5' region of the tomato PG cDNA clone, F1 (Sheehy *et al., Mol. Gen. Genet.* (1987) 208:30-36), at the unique *Xba*I restriction enzyme site. The tag was inserted in the antisense orientation resulting in plasmid pCGN1271 and in the sense orientation yielding plasmid pCGN1270. Plasmids pCGN1270 and pCGN1271 were linearized at the unique *Bgl*II restriction enzyme site and cloned into the binary vector pCGN783 (described above) at the unique *Bam*HI restriction enzyme site to yield pCGN1268 and pCGN1269 respectively.

Preparation of pCGN1219 and pCGN1220

pCGN1273 was tagged with a 0.5 kb fragment of DNA (base number 566 to 1055) from a PG genomic clone which spans the 5' end of the intron/exon junction (see Figure 5). This fragment was cloned into the *Xba*I site resulting in plasmid pCGN1215. pCGN1215 was linearized at the unique BglII site and cloned into pCGN783 at the *Bam*HI site resulting in two plasmids, pCGN1219 and pCGN1220, which differ only in the orientation of pCGN1215 in pCGN783.

Preparation of pCGN1255 and pCGN1258

The 383 bp *Xba*I fragment from the PG cDNA clone was cloned into the unique *Cla*I site of pCGN1267 after filling in the *Xba*I and *Cla*I ends with Klenow and blunt ligation. The fragment in a sense orientation resulted in plasmid pCGN1263 and in the antisense orientation gave pCGN1262. pCGN1263 was linearized at the unique *Bgl*II site and cloned into pCGN783 at the *Bam*HI site yielding pCGN1260. pCGN1262 was also linearized at the *Bgl*II site and cloned into pCGN783 at the *Bam*HI site resulting in two plasmids, pCGN1255 and pCGN1258, which differ only in the orientation of pCGN1262 in the binary vector pCGN783.

Preparation of pCGN1227 and pCGN1228

The 0.5 Kb fragment of the PG genomic clone spanning the intron/exon junction (see Figure 5) was cloned into pCGN1267 at the *Cla*I site in an antisense direction yielding plasmid pCGN1225. This plasmid was linearized at the *Bgl*II restriction enzyme site and cloned into pCGN783 at the *Bam*HI site producing two palsmids, pCGN1227 and pCGN1228, which differ only in the orientation of pCGN1225 in the binary vector.

Preparation of pCGN1264 and pCGN1265

The *Eco*7 fragment (base numbers 5545 to 12,823) (Barker *et al., Plant Mol. Biol.* (1983) 2:335-350) from the octopine plasmid pTiA6 of *Agrobacterium tumefaciens* (Knauf and Nester, *Plasmid* (1982) 8:45-54) was subcloned into pUC19 at the *Eco*RI site resulting in plasmid pCGN71. A *Rsa*I digest of pCGN71 allowed a fragment of DNA from bases 8487 to 9836 of the *Eco*7 fragment to be subcloned into the vector ml3 BlueScript Minus (Stratagene, Inc.) at the *Sma*I site resulting in plasmid pCGN1278. This fragment contains the coding region of the genetic locus designated *tmr* which encodes a dimethylallyl transferase (isopentenyl transferase) (Akiyoshi *et al., Proc. Natl. Acad. Sci. USA* (1984) 81:5994-5998; Barry *et al.,*

(1984) 81:4776-4780). An exonuclease/mung bean treatment (Promega Biotech) of pCGN1278 produced pCGN1272 in which there was a deletion on the 5' end of the *tmr* gene to a point 39 base pairs 5' of the start codon. The *tmr* gene from pCGN1272 was subcloned into the *ClaI* site of pCGN1267. The *tmr* gene, in the sense orientation, yielded plasmid pCGN1266. pCGN1266 was linearized at the *BglII* site and subcloned into pCGN783 at the *BamHI* site yielding two plasmids, pCGN1264 and pCGN1265, which differ only in the orientation of pCGN1266 in pCGN783.


## Example 6


## Preparation of Transgenic Plants


Feeder plates were prepared by pipetting 0.5 ml of an eight day old suspension of *Nicotiana tabacum cv xanthi* cell suspension culture $10^6$ cells/ml) onto 0.8% agar medium, containing MS salts, myo-inositol (100 mg/l), thiamine-HCl (1.3 mg/l), sucrose (30 g/l), potassium acid phosphate (200 mg/l) 2,4-D (0.2 mg/l), and kinetin (0.1 mg/l) (pH 5.5). The feeder cells were prepared at least 24 hours prior to use. A #1 Whatman sterile filter paper (Whatman Ltd. Maidstone, England) was placed on top of the tobacco feeder cells after the cells had been growing for at least 24 hours.

Agrobacteria containing the plasmid of interest were grown on AB medium $K_2HPO_4$, 3 gm/l; $NaH_2PO4.H_2O$, 1.15 g/l; $NH_4Cl$, 1 g/l; KCl 0.159/l; glucose, 5 g/l; $FeSO_4$, 0.25 mg/l; $MgSO_4$, 0.246 mg/l; $CaCl_2$, 0.14 mg/l; 15 g/l agar; gentamicin sulfate, 100 $\mu$g/l; and streptomycin sulfate, 100 $\mu$g/l) for 4-5 days. Single colonies were then inoculated into 5 ml of MG/L broth and preincubated overnight in a shaker (180 rpm) at 30°C.

Sterile tomato cotyledon tissue was obtained from 7-8 day old seedlings which had been grown at 24°C, with a 16hr/ 8hr day/night cycle in 100 x 25 mm petri dishes containing MSSV medium: Murashige-Skoog (MS) salts (#1117 Gibco Laboratories, New York), sucrose 30 g/l, Nitsch vitamins (Thomas, *et al., Appl. Genet.* (1981) 59:215-219), 0.8% agar (pH 6.0). Any tomato species may be used as a tissue source, however, the inbred breeding line UC82B (Department of Vegetable Crops, University of California, Davis) is preferred. The tips and bases of the cotyledons were removed and the center section placed onto a feeder plate for a 24-hour preincubation period in low light, preferably less than 80 microEinsteins, generally about 40-50 microEinsteins, at 24°C.

Following the preincubation period, the cotyledon explants were dipped into an agrobacteria suspension ($5 \times 10^8$ bacteria/ml) for approximately 5 minutes, blotted on sterile paper towels and returned to the original tobacco feeder plates. The explants were cocultivated with the agrobacteria for 48 hours on the tobacco feeder plates in low light (see above) at 24°C, then transferred to regeneration medium containing 500 mg/l of carbenicillin disodium salts and at least 100 mg/l of kanamycin sulfate. The regeneration medium is MS salts medium with zeatin (2 mg/l), myo-inositol (100 mg/l), sucrose (20 g/l), Nitsche vitamins and 0.8% agar (pH 6.0). After 10 days and subsequently every three weeks, the explants were transferred to fresh regeneration medium containing 500 mg/l of carbenicillin disodium salts and at least 100 mg/l of kanamycin sulfate. Shoots were harvested from 8 weeks onwards and placed on MSSV medium containing carbenicillin (50 mg/l), kanamycin (50 mg/l) and indole-3-butyric acid (1 mg/l). Roots developed in 7-14 days. Plants were then transplanted into soil.

An aminoglycoside phosphotransferase enzyme (APH3'II) assay is conducted on putative transformed tomato plants and shoots. APH3'II confers resistance to kanamycin and neomycin. APH3'II activity is assayed (Reiss *et al., Gene* (1984) 30:211-218) employing electrophoretic separation of the enzyme from other interfering proteins and detection of its enzymatic activity by *in situ* phosphorylation of kanamycin. Both kanamycin and [$\lambda^{-32}P$] ATP act as substrates and are embedded in an agarose gel which is placed on top of the polyacrylamide gel containing the proteins. After the enzymatic reaction, the phosphorylated kanamycin is transferred to P-81 phosphocellulose ion exchange paper and the radiolabeled kanamycin is finally visualized by autoradiography. The Reiss *et al.*, method is modified in the final washing of the P-81 ion exchange paper by rinsing in 0.1 mg/ml of proteinase K.


## Example 7


19

## Analysis of Expression Of Tagged Genomic Constructs in Transgenic Plants

Immature green fruit (approximately 3.2 cm in length) were harvested from two tomato plants c. UC82B that had been transformed with a disarmed *Agrobacterium* strain containing pCGN1264. Transgenic plants are designated 1264-1 and 1264-11. The pericarp from two fruits of each plant was ground to a powder under liquid nitrogen, total RNA extracted and polyA$^+$ mRNA isolated as described in Mansson *et al., Mol. Gen. Genet.* (1985) 200:356-361. Young green leaves were also harvested from each plant and polyA$^+$ mRNA isolated .

Approximately 19 μg of total RNA from fruit, 70 ng of polyA$^+$ mRNA from fruit and 70 ng of polyA$^+$ mRNA from leaves from transformed plants 1264-1 and 124-11 were run on a 0.7% agarose formaldehyde Northern gel and blotted onto nitrocellulose (Maniatis *et al., Molecular cloning: A Laboratory Manual -* (1982) Cold Spring Harbor, New York). Also included on the gel, as a negative control, were approximately 50 ng of polyA$^+$ mRNA from leaf and immature green fruit of a nontransformed uC82B plant.

As a positive control, and to help in quantitating mRNA levels, *in vitro* transcribed RNA from pCGN1272 was synthesized using T3 polymerase (Stratagene, Inc.). Nineteen pg and 1.9 pg of this *in vitro* synthesized RNA were loaded onto the Northern gel.

The probe for the Northern filter was the 1.0 kb *tmr* insert DNA (a *Kpn*I to *Sac*I fragment) from pCGN1272 isolated by electroelution from an agarose gel (Maniatis, (1982), *supra*) and labeled by nick translation (Bethesda Research Laboratory kit) using $\alpha^{32}$P dCTP (Amersham).

The Northern filter was prehybridized at 42°C for 5 hrs in the following solution: 25 ml Formamide, 12.5 ml 20X SSC, 2.5 ml 1 M NaP, 5 ml 50X Denharts, 0.5 ml 10% SDS, 1 ml 250 mM EDTA, 1 ml 10 mg/ml ssDNA and 2 ml H$_2$O. Then one-fifth volume of 50% dextran sulfate and approximately 2.2 x 10$^7$ cpm of the probe were added and hybridization was carried out for 15 hr at 42°C. The Northern filter was washed one time in 2X SSC and 0.1% SDS at 55°C and twice in 1X SSC and 0.1% SDS at 55°C for 20 minutes each wash. The filter was allowed to air dry before being placed with Kodak XAR film and an intensifying screen at -70°C for two days.

## Northern Results on Transgenic Plants

The nicked *tmr* probe hybridized with a mRNA species approximately 1.7 Kb in length was observed in the total RNA and polyA$^+$ mRNA fruit lanes of the Northern blot. This is the expected length of the reintroduced 2All gene (0.7 Kb) tagged with the *tmr* gene (1.0 kb). The level of expression from the reintroduced tagged gene is somewhat lower than the level of expression from the endogenous 2All gene. The level of expression of the reintroduced gene in immature green fruit is higher than the expression level in leaf tissue, although there appears to be a small amount of hybridizing mRNA in leaf tissue in these transformants .

## Example 8

## 2All Promoter Cassette

The design of the 2All cassette is shown in Figure 7. The cassette contains 3.8 kb of DNA 5′ of the transcriptional start site and the entire 3′ region (from the TGA stop codon to a site 2.0 kb 3′ of the poly A addition site) of the 2All gene. Figure 7 shows the restriction sites and indicates (below the representation of the gene) the regions of the 2All gene used to construct the 2All cassette. The 2All cassette was constructed as follows.

## Transcriptional Initiation Region

The 5′ end of the 2All cassette was constructed starting with an *Eco*RI subclone genomic clone as described in application PCT/US88/01811 cloned into the *Eco*RI site of Bluescript (+) (Stratagene) resulting in pCGN1288. A map of plasmid pCGN1288 is shown in Figure 6. This clone contains sequences from the *Eco*RI site at position 1651 in the intron of the 2All gene to the *Eco*RI site located 2.5 Kb upstream of the *Xho*I site at position 1 of the sequenced region (see Figure 7). The *Xho*I fragment from position 1 of the sequenced region to the *Xho*I site in the Bluescript polylinker was deleted creating plasmid pCGN2004 which contain the 2All region from position 1 to position 1651. The coding region of 2All was deleted by treating this plasmid with ExonucleaseIII/S1 using the commercially available Erase-a-Base Kit (Promega Biotec) and sequencing deletion plasmids until one was found which had the coding region deleted to position 1366. The resulting plasmid, pCGN1251, had the genomic region from the *Xho*I site (position 1) to position 1366. The *Eco*RI fragment of pCGN1288 was then transferred to a chloramphenicol resistant plasmid vector, pCGN2015, to make pCGN1231. pCGN2015 is a Cm resistant derivative of the Bluescript plasmid. A BstEII/*Bam*HI fragment of pCGN1251 was then transferred into BstEII/*Bam*HI digested pCGN1231 to make pCGN1235 which contains the region from the *Eco*RI site (2.5 kb upstream of the sequenced region) to position 1366 of the sequenced region flanked by the Bluescript polylinker in a Cm resistant vector.

## Transcriptional and Translational Termination Region

The 3′ end of the 2All cassette was constructed from pCGN1273 (described in application PCT/US88/01811) by digesting the plasmid with *Pvu*I and *Eco*RI, isolating the 2249 bp insert (from position 2402 to 4653), ligating with a double-stranded oligonucleotide containing the sequence shown in Figure 7 from the *Bam*HI sticky end to a *Pvu*I sticky end into a Bluescript vector which has been digested with *Bam*HI and *Eco*RI. The resulting plasmid, pCGN1238, contains the 3′ end of the 2All gene from the stop codon at position 2381 to the *Eco*RI site at position 4653.

## Final Construction

Several versions of the 2All cassette in different vectors with different flanking restriction sites have been constructed; maps of the plasmids are shown in Figure 8.

A cassette containing the 5′ and 3′ regions of the 2All gene was constructed by ligating the *Bam*HI to *Eco*RV insert of pCGN1238 into pCGN1235 which had been digested with *Bam*HI and *Xba*I (the *Xba*I site having been filled in with Klenow polymerase to make a blunt-ended fragment). The resulting plasmid, pCGN1240, has the 5′ end of the 2All gene from the *Eco*RI site 2.5 kb upstream of the *Xho*I site (position 1) to position 1366 (which is located between the transcriptional initiation site of the 2All gene and the ATG), followed by a polylinker region (sequence given in Figure 6) with sites for *Sma*I, *Bam*HI, *Pst*I and *Sal*I which can be conveniently used to insert genes followed by the 3′ region from position 2381 to 4653. The plasmid backbone of pCGN1240 is the Bluescript Cm plasmid described above.

## Construction of Plasmid pCGN1241

A more convenient version has the *Eco*RI of pCGN1240 excised and inserted into a Bluescript vector called pCGN1239 which has an altered polylinker region such that the entire cassette can be excised as a SacI-*Kpn*I fragment. The altered BlueScript vector, pCGN1239, was constructed by modifying the BlueScript polybinder from the SacI site to the *Kpn*I site including asynthetic polylinker with the following sequence: AGCTCGGTACCGAATTCGAGCTCGGTAC to create a polylinker with the following sites: SacI-*Kpn*I-*Eco*RI SacI-*Kpn*I. The *Eco*RI insert of pCGN1240 was inserted into pCGN1239 to make pCGN1241 (see Figure 8).

## Construction of pCGN2610 and pCGN2611

A chloramphenicol resistant version of the 2All promoter cassette was constructed by inserting the synthetic polylinker described above (see construction of pCGN1241) into pCGN2015 to make pCGN1246, followed by insertion of the *Eco*RI fragment of pCGN1246, followed by pCGN2610 and pCGN2611 which differ only by the orientation of the inserted fragment in the plasmid vector (see Figure 8).

Example 9

Comparison of different Sized 2All 5′ Regions

A beta-glucuronidase (Gus) reporter gene was used to evaluate the level of expression and tissue specificity of various 2All-Gus constructions. Gus is a useful reporter gene in plant systems because it produces a highly stable enzyme, there is little or no background (endogenous) enzyme activity in plant tissues, and the enzyme is easily assayed using fluorescent or spectrophotometric substrates. See, for example, Jefferson, *Plant Mol. Biol. Rep.* (1988) 5:387-405. Histochemical stains for Gus enzyme activity are also available which can be used to analyze the pattern of enzyme expression in transgenic plants. Jefferson (1988), *supra*.

Constructions containing 1.3 kb (short), 1.8 kb (intermediate length), or 3.8 kg (long) 2All 5′ sequences fused to the Gus reporter gene were prepared. In addition, constructions were prepared which have altered 3′ ends. The altered 3′ ends are either a shorter 2All 3′ end, or a 3′ end from tr5 of the T-DNA of the Ti plasmid (Willmitzer *et al., Embo. J.* (1982) 1:139-146; Willmitzer *et al., Cell* (1983) 42:1045-1056. The constructions were transferred to a binary vector (pCGN1578), and used in *A. tumefaciens* cocultivations The resulting binary was used to transform tomato plants. The transgenic plants obtained were fluorometrically analyzed for Gus enzyme activity.

Preparation of Test Constructs

Constructionof pCGN2601 and pCGN2602

These constructs contain 3.8 kb of 2All 5′-Gus 2.4 kb of 2All 3′ sequence, differing only in the orientation of the 2All/Gus construction with respect to the other elements of the binary vector plasmid (see Figure 9).

The constructs were made by inserting the *Pst*I fragment of pRAJ260 (Jefferson, *supra*) into the *Pst*I site of pCGN1240. The resulting plasmid, having the Gus gene in the sense orientation with respect to the 2All promoter, was named pCGN1242 (Figure 10). The 2All/Gus construction was excised as an *Eco*RI fragment and cloned into the *Eco*RI site of pCGN1239 to make pCGN1247. The insert of pCGN1247 was then excised as a *Kpn*I fragment and cloned into the *Kpn*I site of the binary vector pCGN1578 to make pCGN2601 and pCGN2602. The orientation of the construction within the binary vector seems to have no effect on expression of the DNA sequence of interest.

Construction of pCGN2812

pCGN2800 was constructed be deleting an *Xho*I fragment from pCGN1242. The resulting in a 2All/Gus construction containing 1.3 kb of 2All 5′ sequence and 2.4 kb of 2All 3′ sequence, pCGN2800, was linearized with *Kpn*I and the entire plasmid was cloned into the *Kpn*I site of the binary plasmid pCGN1578 to yield pCGN2812.

Construction of pCGN2816

A construction with 1.8 kb of 2All 5′ sequence and 1.0 kb of 2All 3′ sequence fused to the Gus reporter gene was made by digesting pCGN1242 with *Hind*III and cloning into the *Hind*III site of pCGN1578 to give pCGN2816.

Construction of pCGN2813 and pCGN2814 (2All 5′-Gus-Tr5 3′ Constructs)

Two constructions were made in which the 3' sequence of 2All was replaced with the 3' terminator sequence of Tr5 of the T-DNA region of the Ti plasmid (Willmitzer *et al.*, (1982), *supra*; Willmitzer *et al.*, (1983), *supra*). A *Sau*3A fragment from position 2396 to position 2920 of the T-DNA region of the Ti plasmid (Barker *et al.*, (1983) *supra*) was cloned into the *Bam*HI site of pUC18 (Norrander J. *et al., Gene* - (1983) 26:101-106) to give pCGN9VK. A *Hind*III-*Sal*I fragment of pCGN1242 was inserted into *Hind*III-*Sal*I digested pCGN9VK to give pCGN2801 which has 1.8 kb or 2All 5' sequence and the transcript5 3' sequence fused to the Gus reporter gene. In one case, the 2.0 kb *Hind*III fragment of pCGN1242 was also cloned in the proper orientation to give pCGN2802 which has 3.8 kb of 2All 5' and the transcript5 3' end fused to the Gus reporter gene. These plasmids were each linearized with *Kpn*I and cloned into the *Kpn*I site of pCGN2813 (1.8 kb 2All 5') and pCGN2814 (3.8 kb 2All 5'). pCGN2813 and pCGN2814 are shown schematically in Figure 9.

The completed binaries were used for cocultivation. *Agrobacterium tumefaciens* strain 2760 (also known as LBA4404, Hoekema *et al., Nature* (1983) 303:179-180) were transformed with the binary of interest using the method of Holsters *et al., Mol. Gen. Genet.* (1978) 163:181-187. The transformed binary was then used in the cocultivation of plants. Transgenic plants were prepared as set forth in Example 6.

Analysis of Gus Enzyme Activity

$\beta$-glucuronidase activity was measured using 4-methyl umbelliferyl glucuronide as substrate as outlined in Jefferson, *PMB Reporter* (1987) 5:387-405. In untransformed tomato leaves, the background enzyme levels were 5-10 pmol methyl umbelliferone (MU)/mg protein/min. Using the $\beta$-glucuronidase activity assay, timing of Gus expression under the control of a 3.8 kb 5' 2All sequence was evaluated at various developmental stage of tomato fruit. Gus enzyme activity was first detectable when the fruit began expanding, namely at approximately 13 days post-anthesis. Enzyme activity increased steadily throughout the fruit maturation stages (before ripening).

Gus enzyme activity was not seen in flower parts (unexpanded ovaries, stigma + style tissue, petals, sepals, or anthers) when transformants containing constructs with 3.8 kb of 2All 5' sequence such as pCGN2601 or pCGN2602 were analyzed. Some Gus enzyme activity was observed in leaf tissue, however. Surprisingly, an increased percentage of transformants in which Gus enzyme activity was not expressed in leaf but was expressed in fruit may be obtained through the use of the longer 2All 5' regions. Thus, sequences of up to about 1.3 kb upstream of the 2All promoter gave relatively high levels of expression in fruit but did not show optimal fruit-specific expression of inserted genes. Inclusion of an additional approximately 500 bp (the 1.8 kb 5' to the 2All promoter) increased fruit-specificity but reduced levels of expression observed in the fruit.

Longer promoter constructions (3.8 kb 5' of the gene) gave a higher percentage of transgenic plants expressing the gene at high levels in the fruit as well as tighter control of fruit-specificity. For example, out of 30 pCGN2601/pCGN2602 (2All "long" promoter) transgenic plants analyzed, 27 (90%) showed the highest Gus enzyme activity in fruit and little or no detectable Gus enzyme activity in leaves. One transformant did show Gus enzyme activity in leaf tissue at a level approximately twice background in leaf tissue. The level of enzyme activity in the fruit ranges up to greater than 100,000 pmol MU/mg protein/min. Background Gus enzyme activity in fruit is 50 to 100 pmol/MU/mg protein/min.

Approximately 35% (7/22) of the pCGN2812 (2All "short" promoter) transgenic plants showed high levels of Gus enzyme activity in the fruit, the levels being comparable to the levels observed with the "long", 3.8 kb 2All 5' region. However, 30% (14/24) of the plants containing the pCGN2812 construct also showed low levels of Gus activity in the leaves (levels are from 2X to 20X background leaf levels). Thus, some of the plants expressed Gus in both leaves and fruit, some in fruit alone. None of the plants expressed Gus in leaves alone. The frequency of transgenic plants that expressed the 2All/Gus construct was lower when a shorter 5' end is used (35% vs. 90%) and the specificity of the expression was reduced: more plants were found that had measurable Gus activity in the leaf tissue. However, with the pCGN2812 construct, the level of expression in the fruit of plants that expressed the construct was as high as the levels obtained from the longer promoter.

None of the transgenic plants containing the intermediate length (1.8 kb) 5' end constructs showed any measurable Gus activity in leaves (CGN2813 (0/7); pCGN2816 (0/12)). However, in contrast to the results obtained with either the long (3.8 kb) promoter constructs or the short (1.3 kb) promoter constructs, none of the plants with the intermediate length (1.8 kb) promoter showed high levels of Gus enzyme activity in the fruit [pCGN2813 (0/5), pCGN2816 (0/19)]. The fruit from the pCGN2813 and pCGN2816 plants did show some Gus enzyme activity but the level was low in comparison to the high levels from the pCGN2812 plants.

From the foregoing, it is seen that the length of the 2AII 5′ region can influence the timing, level and tissue specificity of gene expression. The results suggest the influence of negative and positive expression modifiers in regions further upstream than the originally described 1.3 kb 2AII 5′ promoter region.

EXAMPLE 10

Functional Mapping Of The 2AII 5′ Region

Isolation of Nuclei And Nuclear Proteins

Isolation procedure for nuclei was basically as described by Maier, et al, *EMBO J.* (1987) 6:17-22, with some modifications. Leaf or mature green fruit tissue (40 g) was homogenized using the Polytron (10 mm attachment, setting 4) in 100 ml of a buffer containing 2.5% (w/v) Ficoll 4000, 5% ((w/v) Dextran T40, 0.5% Triton X-100, 25 mM Tris (pH = 8.0), 25 mM EDTA, 0.44 M Sucrose, 10 mM $\beta$-mercaptoethanol and 2 mM Spermine. The solution was filtered through four layers of cheese cloth and through two layers of miracloth, followed by a 5 min centrifugation spin at 4000 rpm. The pellet was washed 2-3 times with the same buffer, before resuspending it in 1 ml of a buffer containing 10 mM Tris (pH = 7.9), 5 mM $MgCl_2$, 25% glycerol, 10 mM beta-mercaptoethanol, 5 mM EDTA, 1 mM PMSF and 2 mM DTT. At this point 4 M NaCl was added slowly to the solution, to a final concentration of 0.4 M. The disruption of the nuclei was monitored under a microscope. The solution was centrifugated for 20 minutes at 16000 rpm. The supernatant was then dialyzed against a buffer, containing 20 mM Hepes (pH = 7.9), 12.5 mM MgCl2, 0.2 mM EDTA, 20% glycerol, 100 mM KCl, 1 mM PMSF and 2 mM DTT. The protein content of these samples was measured by the method of Bradford (Bradford, 1976) and the samples were then quick frozen in liquid N2 and stored at -80° C until used in the gel retardation of experiments.

Preparation Of Probe And Competitor DNA

DNA fragments were synthesized using primers and the polymerase chain reaction (PCR), with the 5′-region of the 2AII gene as a template following the manufactures direction (Perkin and Elmer, Cetus). The fragments were labeled during the PCR reaction by adding $^{32}$P dCTP to the reaction. After the reaction the fragments were cleaned by two phenol/chloroform extractions. The unlabeled fragments were obtained exactly the same way as the labeled fragments were obtained but without the use of $^{32}$P-dCTP. The DNA fragments were stored at -20° C.

The coordinates of the fragments used in the different constructs are taking the first base of the mRNA to be +1 (Pear et al ., Plant Mol. Biol. (1989) 13:639-651): pCGN3138 (H) -1025 to -426, pCGN3139 (W) -575 to -276, pCGN3142 (K) -1325 to -739, pCGN 3143 (D) -1025 to -739, pCGN3144 (500) -1805 to -1325, pCGN3147 (5A) -1596 to -1325, pCGN3148 (U) -279 to +40, pCGN3153 (Z) -1177 to -875, pCGN3154 (C) -737 to -426, pCGN3155 (Y) -875 to -576, pCGN3163 (E) -1325 to -1026, pCGN3165 (5B) -1805 to -1595, pCGN3166 (B) -425 to -104 and pCGN3167 (G) -737 to -104. Other fragments used in the gel retardation experiments: fragment F -425 to +40 and fragment 5 A T -1596 to -1174. Fragment L, containing the poly-linker region of the pIC19R plasmid (Marsh, et al., *Gene* (1984) 32:481-484) was obtained by a PCR-reaction with the universal and reverse primer and the pIC19R plasmid as a template.

Gel Retardation Experiments

For the gel retardation experiment a fresh 10X binding buffer described by Green, et al., *EMBO J.* - (1987) 6:2543-2549, was used. The binding reaction (30 ul) would typically consist of 3 ul of 10x buffer, 3-5 ul of labeled DNA fragment (100 ng), 1 ul poly dI/dC DNA, and 1 to 3 ug of protein extract in an end volume of 30 ul. In competition experiments a 5-fold excess of cold fragment (1 ug) was added to the reaction in the pre-incubation stage.

Binding occurred for 10 min at room temperature without the labeled fragment, followed by 15 min room temperature with the labeled fragment added. The samples were then directly loaded on a pre-run polyacrylamide gel (3.5 to 5.0%) with 0.5x TBE as a buffer, under constant circulation of the buffer during the run.

## Construction Of Truncated CaMV 35S-Cassette

The truncated CaMV 35S promoter cassette was constructed by insertion of an *EcoRV* (-90)/*Bam*HI (+113) fragment of the CaMV 35S 5-region of strain CM1841 in front of the Gus coding region with a 927 bp 2All 3'-region in a pUC8 Plasmid. This plasmid still contains a unique *Eco*RI site directly in front of the *Eco*RV site of the CaMV 35S 5'-region. All tested fragments of the 2All 5'-region were obtained using the polymerase chain reaction with primers containing convenient restriction sites at the ends, so that these PCR-fragments could be cloned into the pIC19R vector. This allowed these fragments to be retreived from the vector as *Eco*RI fragments, and subsequently inserted in the truncated 35S promoter cassette. The 2All/35S-gus-2All gene was then inserted as a *Hind*III fragment into the binary vector pCGN1578, which contains a 35S-neomycin-tml gene for the selection of transformed plantlets (McBride and Summerfelt, 1990).

All fragments obtained with the PCR method were sequenced to verify the proper nucleotide composition as available in the database under nr, xxxx. Using the method of Sanger, et al., *Proc. Natl. Acad. Sci. USA* (1977) 74:5463-5467, only two base alterations could be found due to a mistake inserted by the Taq-polymerase. Fragment D contains a base substitution oat position 3924, changing an A to a T. Fragment Z contains a base substitution as position 3707, changing a T to a G.

## Plant Transformation

The binary vector was introduced in *Agrobacterium tumefaciens, strain* LBA4404 (Hoekema, et al., *Nature* (1983) 303:179-181) for cocultivation with *Lycopersicon esculentum.* Hypocotyls of Tomato UC82B seedlings were cocultivated as described in Fillatti, et al., *Bio/Technology* (1987) 5:726-730, with modifications as described in McBride and Summerfelt, *Plant Mol. Biol.* (1990) 14:269-276.

## Kanamycin And Gus Assays

Kanamycin Radke et al., Theor. App. Genet. (1988) 75:685-691 and Gus assays (Jefferson, Plant Mol. Biol. Rep. 5:387-405) were performed as previously. To study the elements involved in the activity of the 2All 5' region, the 5' region was divided into fragments as described above. Coordinates of the 2All 5' - fragments used are the distance from the mRNA start site (-53 from the translational start codon). These fragments were used separately in both activity and protein binding studies. Introduction of these fragments in front of a truncated CaMV 35S promoter, revealed the presence of a large number of positive enhancer elements within the 2All 5'-region, of which a number induced a fruit-specific enhancement. A functional map of the 2All 5'-region indicating the positions of various cis-acting elements relative to each other was obtained as follows.

## Binding Of Nucleoproteins To Sequences In The 2All 5'-region

Fragments of the 2All 5'-region were labelled during a PCR reaction with $^{32}$P dCTP and submitted to gel retardation experiments using nucleoprotein extracts of both leaf and mature green fruit tissue obtained as described above. A schematic of the fragments is shown in Fig. 11. Results of the gel retardation experiments are shown in Figures 12 A through C. As shown in Figure 12A, the 481 bp fragment 500 shows a mobility shift due to protein binding after incubation with both leaf and fruit protein extracts whereas the 587 bp fragment K shows a mobility shift only when incubated with fruit nuclear proteins. Fragments 5A and 5B which together make up fragment 500, also show a definite shift after incubation with fruit extracts. Fragment 5A also shows a shift when incubated with leaf extract. Fragment 5B was ambiguous in this experiment; however, in subsequent experiments it was shown that the binding of fragment 5B to leaf extract proteins was not specific; non-specific DNA sequences competed for this binding.

In Figures 12B and 12 C are shown competition experiments for binding of fragments G and K. As shown in Figure 12B, fragment G binds fruit nuclear proteins; a ten-fold excess of unlabelled fragment G competes for this binding. Unlabelled fragments K, 5At and F do not completely compete with the binding for fragment G whereas unlabelled fragment H clearly competes for the binding. Fragment H overlaps fragment G over a 312 bp region. A similar experiment with labelled fragment K is shown in Figure 12C. With fruit protein extracts there is a clear band shift whereas with leaf extracts only a small shift is present, which is non-specific (data not shown). Fragment G competes for the fruit-specific binding. However, fragments D and E which together make up fragment K do not significantly compete for the binding to fragment K.

Table II represented the results of at least three reproductions for each experiment. All tested fragments showed the ability to bind a fruit-specific protein. Specificity was determined by competition with an excess of the cold fragment itself. The labelled fragment used in each experiment as shown in Column 1; the cold fragment used in excess to determine specificity of binding is shown in Column 5. As shown, only fragments C and E competed for binding to both fragment C and fragment E. Fragments G, H and K all competed for binding to fragment K. Non-specific DNA fragments were not able to compete for binding to these fragments and fruit-specific binding could not be detected when a labeled non-specific fragment, fragment L, (consisting of the poly linker of plc19R) was used in the binding reactions.

Some leaf protein induced retardation could be detected with the larger fragments G, K and H but these results were not always reproducible. The binding of leaf proteins to fragments 500 and 5A was clear and reproducible. The binding sites of the different proteins, based upon these results have been mapped as shown in Figure 13. Based upon these results, three fruit-specific binding sites were identified in the 5′ - region extending from the mRNA start site through to -1805. Additionally, one general binding factor site was identified. This information is summarized in Figure 13.

TABLE II

|  | Column 1 | Column 2 | Column 3 | Column 4 | Column 5 |
|---|---|---|---|---|---|
| 1 | fragment | coordinates | nuclear extract | nuclear extract | competed by |
| 2 |  |  | leaf | fruit |  |
| 3 | G | -737 to -104 | +/- | + | G/H |
| 4 | H | -1025 to -426 | +/- | + | G/K |
| 5 | K | -1325 to -739 | +/- | + | G/H/K |
| 6 | 500 | -1805 to -1325 | + | + | 500 |
| 7 | B | -426 to -104 | - | + | B |
| 8 | C | -737 to -426 | - | + | C/E |
| 9 | D | -1025 to -738 | - | + | D |
| 10 | E | -1325 to -1028 | - | + | C/E |
| 11 | 5A | -1596 to -1325 | + | + | 5A |
| 12 | 5B | -1805 to -1595 | - | + | 5B |
| 13 | L |  | - | - | - |

## Enhancer Properties Of The 2All 5′-region Fragments

The fragments used in the gel retardation experiments and some extra overlapping fragments were inserted in front of the truncated CaMV 35S (-90) promoter to test their ability to promote the expression of the Gus reporter gene in a tissue specific manner.

## Negative Controls

The truncated CaMV 35S (-90) promoter was used as the negative control since it has been shown that this truncated promoter contains elements which can interact synergistically with enhancer elements inserted in front of it (see Benfey, et al., *EMBO J.* (1990) 9:1685-1696 and Poulsen and Chua, *Mol. Gen. Genet.* (1988) 214:16-23). The truncated promoter has been shown to promote low levels of expression in root tissue of transgenic tobacco plants (see Benfey, et al. *EMBO J.* (1989) 8:2195-2202). In tomato plants, using the pCGN3140 comprising the Gus reporter gene, variable expression was observed with both leaf and fruit tissue. Their variability in expression is apparently due to the site of tDNA integration into the plant genome. This level of expression was taken as the background expression level.

Positive Controls

As a positive control, a plant containing a Gus gene driven by a double 35S promoter enhanced mas promoter was used. The double 35S promoter enhanced mas promoter is reported to be at least ten times more active than the CaMV 35S promoter. The Gus activity measured in leaf and fruit tissue of this plant was used as a standardized point of reference for all experiments. Further, as a positive control for the ability of the truncated promoter construct to mediate the enhancement of a particular enhancer fragment, the 35S enhancer (-360 to -90) was reinserted in front of the truncated promoter, creating a small linker at the *Eco*RV-site (pCGN3141). Surprisingly, the resulting promoter is about five times stronger than the double 35S promoter enhanced mas promoter. Activites measuring up to five times higher in leaf and up to eight times higher in fruit tissues than in the positive control plant containing the double 35S promoter enhanced mas promoter in front of the Gus gene were obtained. The double 35S promoter is about ten times more active than the intact CaMV 35S promoter (-941 to +3) this means that the CaMV 35S promoter construct present in pCGN3141 is about fifty times more active than the CaMV 35S promoter. The only differences between this newly constructed promoter and the CaMV 35S promoter are the longer 5'- untranslated region (up to +113) and a shorter enhancer element (-90 to -360 as compared to -90 to -941). Also, the reinsertion of the enhancer element created a small linker (*Xba*1/*Eco*Rv/*Cla*1/*Eco*R1) at the *Eco*RV-site which is part of the pIC19R poly-linker region, that might influence the promoter activity. Similar high levels of expression were obtained when the same enhancer element was cloned in front of the "silent" 1.8 Kbp 2All 5'-region, resulting in the reactivation of this promoter in both leaf and fruit tissues. Therefore the specific fragment of the CaMV 35S 5'-region may contain a very strong enhancer element.

Fragments Of Bp 481-634 (Fragments G, H, K and 500)

The 1.3 Kbp 5'-region can direct normal expression of the Gus gene. This region was divided into fragments G and K which were then individually inserted in front of the truncated CaMV 35S promoter. Fragment G resulted in high levels of expression that were strongly fruit-specific. Insertion of fragment K resulted in a much lower level of enhancement an gave expression in both leaf and fruit, although fruit was higher than leaf. The overlapping 600 bp fragment H showed less ability to enhance the truncated CaMV 35S promoter in leaf tissue, however the enhancement was more pronounced in fruit-tissue. The 481 bp fragment 500, located upstream of the 1.3 Kbp promoter and part of the "silent" 2All promoter, induced Gus expression as a level barely above background, except of in one plant (pCGN3144-10). This observation is consistent with results which indicate that a silencer element is present in this region.

322 And 312 Bp Sub-fragments Of Fragment G

To examine where the enhancer elements were located within the 481-634 bp fragments, and also to determine whether more than one element was involved in the pattern of expression observed, smaller fragments were inserted into the truncated CaMV 35S cassette. Fragments B and C, 322 and 312 base pair fragments, both seemed to contain at least one fruit-specific enhancer element, which together made up the very strong fruit-specific enhancement observed with fragment G. More expression was seen with fragment C than with fragment B. Fragment C, like fragment G, also enhances expression in leaf tissue, therefore a leaf specific or general enhancer may also be present in this fragment. Interestingly, the 300 bp fragment W which overlaps fragments B and C does not induce enhanced expression of the Gus gene in either leaf or fruit tissue when inserted in front of the truncated CaMV 35S promoter. Based upon these results, fragment W either does not contain any enhancer elements, or it contains more than one element wherein the elements have opposing effects. The 320 bp fragment U which contains the TATA-box and transcriptional start site of the 2All gene does not result in enhancement of the truncated 35S promoter above background. This result suggests that the enhancer element present in fragment B is located at the junction between fragments U and W (see Fig. 5).

287 And 300 Bp Sub-fragments Of Fragment K

Fragment K was divided into two fragments, fragments D and E which were then inserted into the truncated CaMV 35S cassette. Fragment D did not significantly induce the expression of the Gus reporter gene above background in either leaf or fruit tissue. This result suggests the presence of a silencer element in this region which would also explain the lower Gus activities obtained in plants containing construct pCGN3139 (fragment H) as compared to plants containing construct pCGN3154 (fragment C).

Fragment E induces a strong fruit-specific enhancement of expression of the Gus reporter gene. This result implies that fragment K contains a fruit-specific element in the region overlapping fragment E and also that a leaf specific or general enhancer element must be present in the region overlapping fragment D or at the junction between fragments D and E. Since the overlapping fragment Z also induces fruit-specific enhancement of the truncated CaMV 35S promoter a strong fruit-specific element must be present in the overlap between fragments Z and E and a general or leaf specific enhancer element found in fragment K must be present within fragment D. Interestingly, fragment Y which overlaps the junctions between fragments G and K induces enhancement of the truncated CaMV 35S promoter in both leaf and fruit tissue therefore a general or leaf specific enhancer must be present in the overlap between fragments D and Y. In addition, fragment D must also contain a silencer element which antagonizes the effects of the enhancer element.

211 and 272 Bp Sub-fragments Of Fragment 500

Fragment 500 when inserted in front of the truncated CaMV 35S promoter had little effect on expression. This fragment was divided into 272 bp fragment 5A and 211 bp fragment 5B. The smaller fragments induced enhancement of the truncated promoter. Fragments 5A induced high levels of Gus expression in leaf tissue and in fruit tissue while fragment 5B induced a clearly fruit specific expression of the Gus gene. Based upon these results, at least one fruit-specific enhancer element is present within fragment 500 as is a general or leaf specific enhancer element. Since fragment 500 itself does not induce a high level of expression of the Gus gene, a strong silencer element must also be present in this region.

The above results are summarized in Fig. 15. Based upon the level and type of expression induced by the various fragments following insertion in front of the truncated CaMV 35S promoter, it is concluded that at least four fruit-specific and three general or leaf-specific enhancer elements are located in the first 1800 bp of the 2All promoter. In Fig. 16, a map of the 2All 5′-region is drawn indicating the relative positions of the fruit-specific enhancer elements, the leaf specific or general enhancer elements and the putative silencer elements. Upstream of the HindIII site at position -1825, a fruit-specific enhancer element is included because the 1.8 Kbp "silent" promoter could be reactivated by the CaMV 35S enhancer in a non-tissue specific manner. This implies that the native 2All promoter harbors at least one fruit-specific enhancer element in the region upstream of the HindIII 3′ site. A more detailed analysis of this upstream region between -1825 and -3824 is needed to determine how many cis-acting elements are located in this region and how far upstream of the transcription start site these elements are positioned.

Comparison of the protein binding results with the map in Fig. 16 suggests that the fruit-specific factor that binds to fragment B may possibly bind to enhancer element F-1. Also the fruit specific factors that bind to fragments C, E and 5B may be binding to enhancer elements F-2, F-3 and F-4 respectively. The general factor binding to fragment 5A may be binding the enhancer element E3. The fruit-specific factor that bound to fragment D may bind either to the silencer element located in fragment D or to an enhancer element present in fragment D which could not be detected due to the presence of the silencer element.

Fragment G induces the most dramatic enhancement of the truncated CaMV 35S promoter. Since this region contains strong fruit-specific enhancer elements, this suggests that the 2All 5′-region contains all necessary information for fruit-specific expression in the first 737 bp or less. Regulatory elements upstream of this region may modulate the expression of this basic 2All promoter region to ensure a more stable and better regulated expression pattern.

The above results demonstrate the ability to identify developmentally regulated sequences in a plant genome, isolate the sequences and manipulate them. In this way, the production of transcription cassettes and expression cassettes can be produced which allow for differentiated cell production of the desired product. Thus, the phenotype of a particular plant part may be modified, without requiring that the regulated product be produced in all tissues, which may result in various adverse effects on the growth, health, and production capabilities of the plant. Particularly, fruit-specific transcription initiation capability is provided for modifying the phenotypic properties of a variety of fruits enhance properties of interest such as processing,

28

organoleptic properties, storage, yield, or the like.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced.

## Claims

1. A DNA construct comprising:
in the direction of transcription, a 2All transcription initiation region comprising at least about 1.8 kb of DNA immediately 5' of the 2All transcriptional start site joined to a DNA sequence of interest other than the wild-type 2All sequence associated with said initiation region, wherein said DNA sequence of interest is under the transcriptional regulation of said initiation region, and a transcriptional termination region.

2. The DNA construct according to Claim 1, wherein said transcriptional initiation region is detectable at least substantially in fruit tissue.

3. The DNA construct according to Claim 1, wherein said 2All transcriptional initiation region comprises at least about 3.8 kb of DNA immediately 5' of the 2All transcriptional start site.

4. The DNA construct according to Claim 1, wherein said transcriptional initiation region is the 2All region.

5. The DNA construct according to Claim 1, herein said DNA sequence of interest is a sequence complementary to a native plant transcript.

6. The DNA construct according to Claim 1, herein said DNA sequence of interest is an open reading frame encoding an amino acid sequence of interest.

7. The DNA construct according to Claim 1, further comprising at least the right T-DNA border of a plant transmissible vector.

8. A plant comprising:
a DNA construct comprising in the direction of transcription, a 2All transcription initiation region comprising at least about 1.8 kb of DNA immediately 5' of the 2All transcriptional start site joined to a DNA sequence of interest other than the wild-type 2All sequence associated with said initiation region, wherein said DNA sequence of interest is under the transcriptional regulation of said initiation region, and a transcriptional termination region.

9. A plant cell comprising:
a DNA construct comprising in the direction of transcription, a 2All transcription initiation region comprising at least about 1.8 kb of DNA immediately 5' of the 2All transcriptional start site joined to a DNA sequence of interest other than the wild-type 2All sequence associated with said initiation region, wherein said DNA sequence of interest is under the transcriptional regulation of said initiation region, and a transcriptional termination region.

European
Patent Office

**EUROPEAN SEARCH
REPORT**

Application Number

**EP 90 30 7915**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 809 334  (CALGENE)<br>* Examples 5-8 *<br>– – – | 1-9 | C 12 N 15/29<br>C 12 N 15/82<br>A 01 H 5/00 |
| P,X | WO-A-8 912 386  (CALGENE)<br>* Examples 5,6,9 *<br>– – – – – | 1-9 | C 12 N 5/10 |

TECHNICAL FIELDS
SEARCHED (Int. Cl.5)

C 12 N
A 01 H

The present search report has been drawn up for all claims

| Place of search | Date of completion of search | Examiner |
|---|---|---|
| The Hague | 17 October 90 | MADDOX A.D. |